(19) Europäisches Patentamt
European Patent Office
Office européen des brevets

(11) Publication number: **0 274 467 B1**

(12) **EUROPEAN PATENT SPECIFICATION**

(45) Date of publication of patent specification: **20.05.92** (51) Int. Cl.5: **A61K 49/00**

(21) Application number: **86904629.2**

(22) Date of filing: **10.07.86**

(86) International application number:
**PCT/US86/01421**

(87) International publication number:
**WO 88/00474 (28.01.88 88/03)**

# (54) HEPATOCYTE DIRECTED VESICLE DELIVERY SYSTEM.

(43) Date of publication of application:
**20.07.88 Bulletin 88/29**

(45) Publication of the grant of the patent:
**20.05.92 Bulletin 92/21**

(84) Designated Contracting States:
**DE FR GB IT**

(56) References cited:
**EP-A- 0 036 277** **WO-A-86/04232**
**US-A- 4 091 088** **US-A- 4 310 505**
**US-A- 4 316 883** **US-A- 4 318 898**
**US-A- 4 377 567** **US-A- 4 544 545**

**Annual Review of Physiology 43 (1981), 172**

**J. Biol. Chem. 246 (1971), 1461-1467**

**J. Biol. Chem. 247 (1972), 4633-4640**

**J. Biol. Chem. 251 (1976), 1296-1302**

**Römpps Chemie-Lexicon, 8th ed., 1983, page 2830**

(73) Proprietor: **GEHO, W., Blair**
**533 Beechwood Street**
**Wooster, OH 44691(US)**

Proprietor: **LAU, John R.**
**1634 Morgan Street**
**Wooster, OH 44691(US)**

(72) Inventor: **GEHO, W., Blair**
**533 Beechwood Street**
**Wooster, OH 44691(US)**
Inventor: **LAU, John R.**
**1634 Morgan Street**
**Wooster, OH 44691(US)**

(74) Representative: **Patentanwälte Beetz sen. - Beetz jun. Timpe - Siegfried - Schmitt-Fumian- Mayr**
**Steinsdorfstrasse 10**
**W-8000 München 22(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 274 467 B1

## Description

The invention relates to compositions comprising hepatocyte-directed vesicles (liposomes) enclosing biological, pharmaceutical or diagnostic materials as a chemically structured delivery system for specific targeting of hepatocytes.

The liver is the human body's largest gland and, as such, receives a massive blood supply through both the portal vein and the hepatic artery. The liver is composed of two types of cells: Hepatocytes or metabolic cells, and reticuloendothelial cells (RE cells) which are scavenger cells. Metabolically, the liver is the most complex organ in the human body and, among other multiple functions, it metabolizes/distributes drugs which are introduced into the organism. The liver is also a target organ for pharmacologically-active agents produced within the body. Accordingly, attempts have been made to provide means for preferentially delivering drugs to the liver for allowing the drug to be handled by the body in a bore natural fashion, thereby improving the drug therapy.

US-A-4,377,567 discloses the use of digalactosyl derivatives such as digalactosyl diglyceride (DGDG) to target vesicles containing drugs, hormones or other biological and diagnostic materials to the liver.

According to this prior art, the liver (hepatocyte) treatment with liposomes has generally been accomplished by accessing the Ashwell receptors of hepatocytes by galactosyl targeting molecules.

The Ashwell receptors of hepatocytes specifically recognize terminal galactose residues (Annual Review of Physiology 43 (1981) 172; J. Biol. Chem. 246, No. 5 (1971) 1461-1467, ibid. 247, No. 14 (1972) 4633-4640, and ibid. 251, No. 5 (1976) 1296-1302).

Examples for appropriate sugar derivatives that can be employed for specific hepatocyte targeting via the Ashwell receptors are

1)

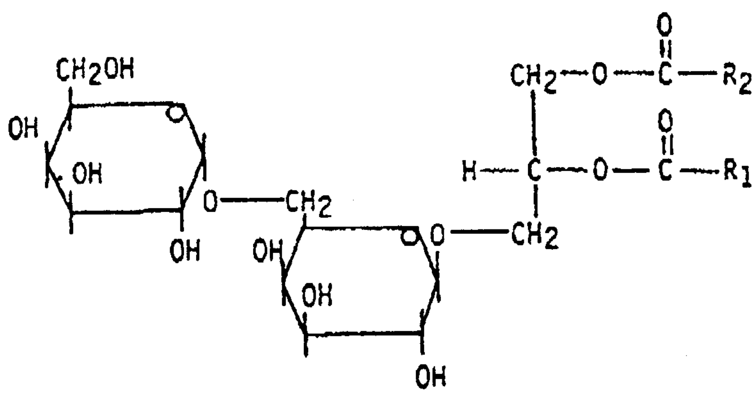

Galactose-α-1-6-galactose-ß-1-1'-diglyceride (DGDG),

2)

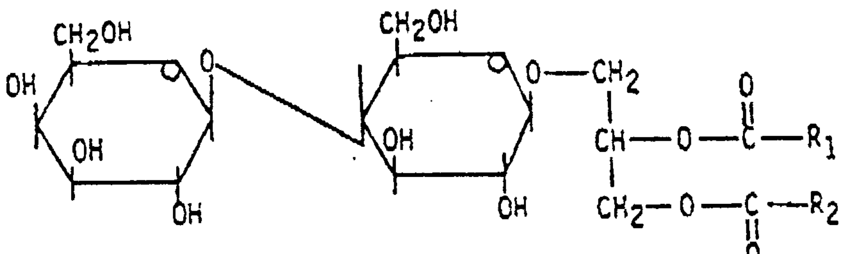

Galactose-ß-1-4-glucose-ß-1-1'-diglyceride,

lactose

and

3)

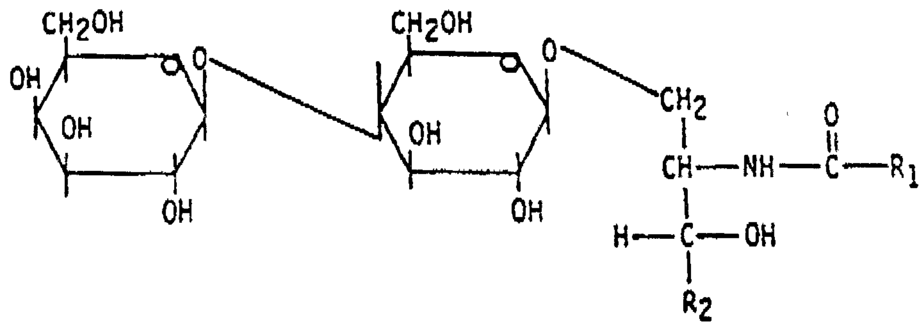

Galactose-β-1-4-glucose-β-1-1'-ceramide,

lactose

i.e. cytolipin H.

This invention is based primarily on the finding that bipolar lipid vesicles containing e.g. a pharmaceutical load can be directed to the hepatobiliary receptors of a liver. It has been found that instead of passing through to the biliary system as expected, vesicles directed to the hepatobiliary receptors will be retained by the hepatocyte with exceptional efficiency.

It is, therefore, the object of the invention to successfully and properly construct hepatocyte directed vesicles (HDV) directed to the hepatobiliary receptors, particularly to enhance the efficiency of liver medication by accessing the hepatobiliary receptors of the liver, and to provide corresponding compositions. Further, the efficiency of hepatocyte directed vesicles in general, shall be enhanced by projection of the target molecule away from the surface of the vesicle.

The above object is achieved according to the claims. The dependent claims relate to preferred embodiments.

The compositions of the present invention consist of or comprise hepatocyte-directed vesicles consisting of a bi-polar lipid membrane enclosing one or more biological, pharmaceutical or diagnostic chemicals and having attached to the lipid membrane targeting molecules extending away from the vesicle; they are characterized in that the targeting molecules have biological specifity for the hepatobiliary receptors of hepatocytes.

According to a preferred embodiment, the targeting molecules comprise a lipophilic end attached to the membrane structure and a targeting portion having biological specifity for the hepatobiliary receptors of hepatocytes.

The targeting molecule preferably have a targeting portion of the formula

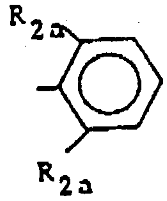

,

wherein $R_{2a}$ is $CH_3$, $CH_3$-$CH_2$ or $CH(CH_3)_2$,
and more preferably of the formula

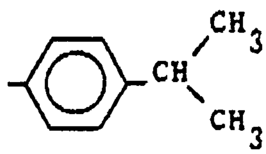

.

In accordance with another preferred embodiment, the targeting molecule is composed of a connector molecule having a lipophilic end attached to the membrane structure and a target molecule comprising the targeting portion, the connector molecule and the target molecule being linked together either directly or by means of a bridge.

Preferably, the connector molecule and the target molecule comprise hydrophilic charged terminal ends linked together by means of a chromium, cobalt, iron or zinc ion.

According to yet another preferred embodiment, the connector molecule and the target molecule are linked together by means of a bridge of the formula

$H_2N$-$CH_2$-$(CH_2)_n$-$NH_2$,

wherein n is 1-4.

In accordance with a further preferred embodiment, the target molecule has for formula

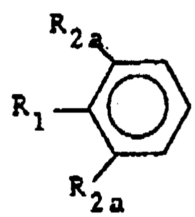

or the formula

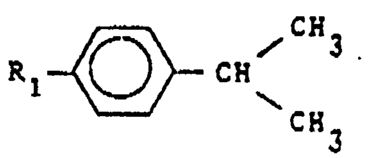

,

wherein $R_1$ is

$$[HOOC-(CH_2)_n-]_2\ N-CH_2-CO-NH-$$

n being 1-3, and
$R_{2a}$ is $CH_3$, $CH_3$-$CH_2$ or $CH(CH_3)_2$.

The targeting molecule may also preferably have the formula

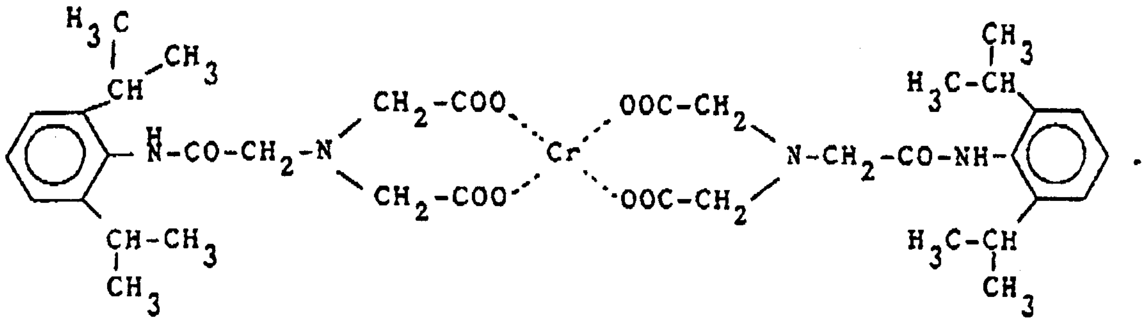

The target molecule and/or the connector molecule may also have the formula

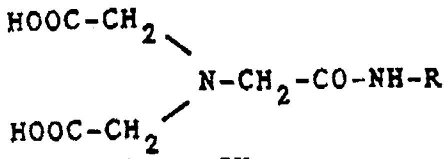,

wherein R is 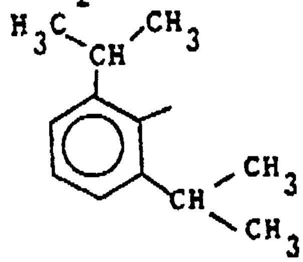, 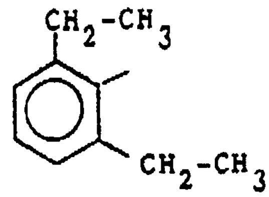,

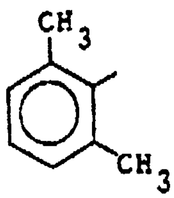, 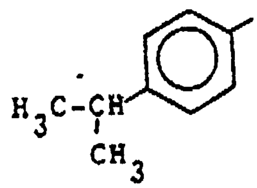, 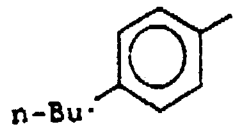,

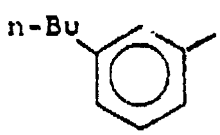, 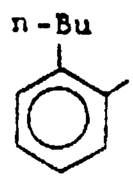, 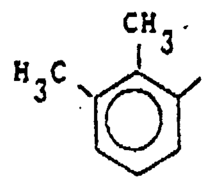,

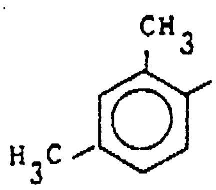, 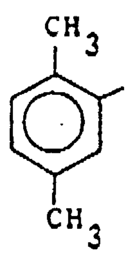, 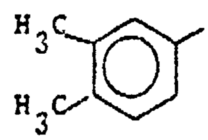,

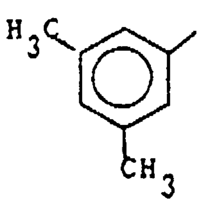, 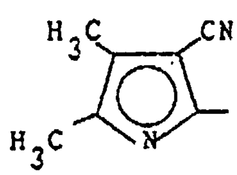, 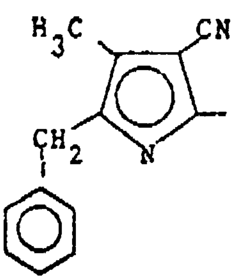,

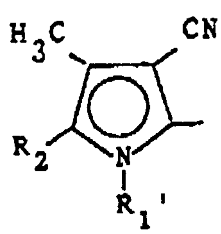,

| $R_1'$ | $R_2$ |
|---|---|
| H | iso-$C_4H_9$ |
| H | $CH_2$-$CH_2$-S-$CH_3$ |
| H | $CH_2$-$C_6$-$H_4$-p-OH |
| $CH_3$ | $CH_3$ |
| $CH_3$ | iso-$C_4H_9$ |
| $CH_3$ | $CH_2$-$CH_2$-S-$CH_3$ |
| $CH_3$ | $C_6H_5$ |
| $CH_3$ | $CH_2C_6H_5$ |
| $CH_3$ | $CH_2C_6H_4$-p-O-$CH_3$, |

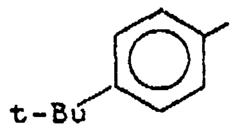 , 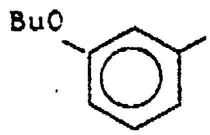 , 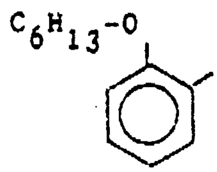 ,

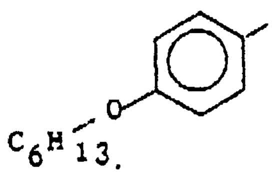 or 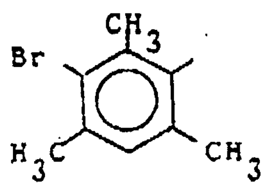

or the formula

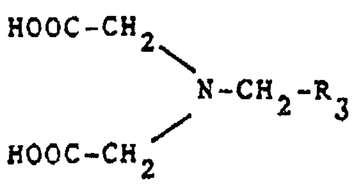 ,

wherein $R_3$ is

$CH_2-NH_2$, 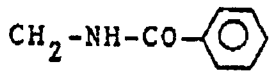 ,

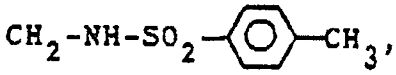

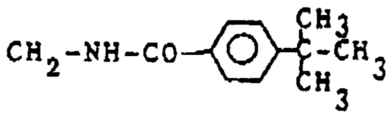 ,

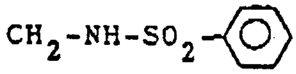 ,

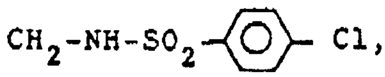

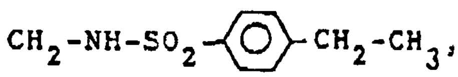

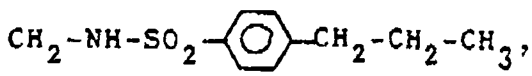

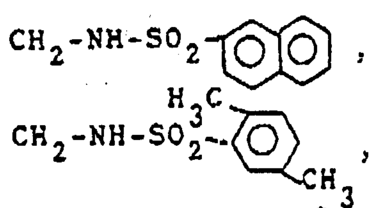 ,

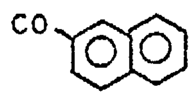 or 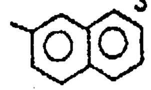 .

In accordance with still another preferred embodiment, the pharmaceutical chemical is insulin.

In the hepatocyte delivery system of the invention comprising a composite of a vesicle and hepatobiliary targeting molecules the vesicle's pharmacologic cargo is detached from its targeting system, and will be retained by the liver cell where it can carry out its pharmacologic action.

The outstanding characteristic of the invention is that the hepatocyte delivery vesicles directed to the hepatobiliary receptors of the hepatocytes not merely pass through to the biliary system, but the cargo is retained by the hepatocytes of the liver, and enabled to carry out its pharmacologic action.

The vesicle structure prevents contact of a pharmacological cargo with cells and tissues of the body which are not intended targets, thereby enhancing the specificity and potency of the pharmacological cargo, and reducing its nonspecific toxicity.

The compositions of the invention can be used for intraduodenal (oral), intravenous, subcutaneous and intramuscular administration.

US-A-4,091,088 relates to assessing the hepatobiliary function by a reagent which is labeled with technetium 0.99 m for use as a hepatobiliary imaging radiopharmaceutical. This material does not affect the liver other than to provide scanning capability for diagnoses.

US-A-4,318,898 and 4,350,674 also relates to assessing the hapatobiliary receptors to cause a quick passage to the biliary system for diagnostic, not treatment, function.

According to GB-A-1,545,427 the hepatobiliary system is also used to diagnose the biliary system.

The subject-matter of this invention distinguishes over the prior art basically in that:

a) the hepatobiliary receptor targeting vesicles will not be merely passed to the biliary system, as would be expected from prior art teaching;

b) the hepatobiliary receptor targeting vesicles can be utilized to deliver hormones and drugs to the liver, particularly insulin.

The means whereby the liver handles insulin illustrates the activity of this important target organ.

Insulin is a hormone which affects the metabolism of the animal as a whole. The most dramatic effect of this hormone is its ability to reduce the concentration of glucose in plasma. Ingested carbohydrate meals are normally digested to glucose in the gut and then absorbed in the portal circulation. The pancreas responds to carbohydrate in the gut with a release of insulin into the portal circulation. The portal vein carries the absorbed glucose and the released insulin to the liver. At the liver the insulin regulates the metabolism of glucose by the hepatocytes. By an unknown mechanism the liver retains most of the insulin but releases some to facilitate glucose utilization by muscle and adipose tissue. Reduction in blood glucose is due to the insulin effect on both liver and peripheral tissues. Thus, while the pancreas is the source of insulin within the organism, the liver is the key to its normal utilization.

Insulin therapy for diabetes mellitus began in 1922. In current medical practice insulin is administered subcutaneously because the oral administration of the insulin is inefficient, presumably due to proteolysis. Subcutaneously administered insulin does produce a lowered level of blood glucose, primarily as a result of its action on muscle and fat tissue. However, insulin administration by injection can hardly be classified as a near normal state. Importantly, the anatomic arrangement of the pancreas in the normal individual is such that high levels of insulin secreted by the pancreas in response to oral glucose loads pass by way of the portal circulation to the liver before entering the peripheral circulation. By comparison, when insulin is administered subcutaneously to the diabetic patient, the peripheral tissue has first access to the hormone and may reduce the level of insulin presented to the liver and, in turn, reduces the effectiveness of the liver as a significant glucose regulating mechanism. Therefore, insulin administered by injection does not have the same physiological action as insulin released from the pancreas.

The present invention provides improved means whereby insulin or other pharmacologically-active agents can be delivered preferentially to the liver in a human or lower animal.

The Lipid Membrane Structures (LMS) used herein comprise a bipolar lipid and a targeting molecule conjugate.

The preferred polar lipid used in the practice of this invention is distearoyl lecithin. Natural lecithin (phosphatidyl choline; vitellin) comprises a mixture of the diglycerides of stearic, palmitic and oleic acids linked to the choline ester of phosphoric acid and is found in all living plants and animals. Vesicles from such polar lipids are known art.

The smaller "liposome" is generally referred to by the term "vesicle", but both are interchangeable for the purposes of this disclosure. The vesicle may be unilammelar or multilamellar and range in size from 250 Å (25 µm) to 5 µm in diameter.

The HDV is useful in delivering drugs, hormones, biologicals or diagnostic materials to the liver. The liver is a difficult organ to reach with exogenously administered drugs, and the HDV presents a unique therapeutic advance, making it possible to deliver drugs, hormones, biologicals and diagnostic materials in a more efficient, safer way than those means currently available. The reason for the "therapeutic unavailability" of the liver to conventional therapies is that the liver is anatomically situated in such a way that it is isolated from the rest of the body with respect to its blood circulation. The majority of the liver's blood comes to it by way of the portal circulatory system which is a highly localized, low-pressure, venous system designed to carry absorbed nutrients (products of digestion) from the intestines to the liver for metabolism. The arterial blood supply takes care of the rest of the body before a small portion of it reaches the liver via the portal system.

In view of the fact that the liver is a difficult organ to reach with exogenously administered drugs, this invention provides a biological carrier system that utilizes hollow bipolar liposomes in conjunction with a family of liver molecules to effect delivery of the prescribed drug.

The following diagram schematically sets forth the basic structure of a functional HDV:

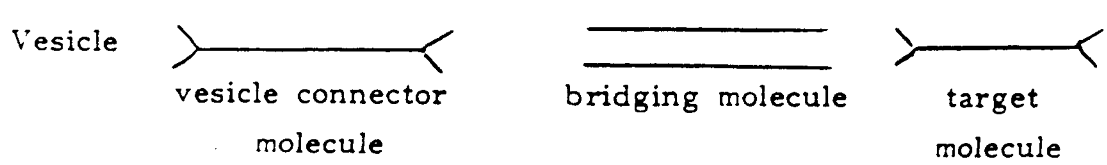

The following structure depicts the basic liposome or bipolar lipid vesicle:

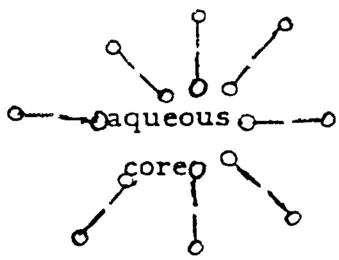


where —o is a polar lipid, — representing a lipophilic portion, and o representing the hydrophilic portion.

The vesicle is a sphere with an aqueous core, where the sphere is a bipolar lipid membrane with hydrophilic surfaces and a lipophilic (hydrophobic) interior of the membrane. The aqueous core can contain water soluble substances such as drugs, hormones, minerals, diagnostic agents, and biologicals. Lipophilic substances are not carried in the core volume but in the bipolar lipid membranes.

The targeting mechanisms require that the molecular target molecule be positioned on the surface of the liposome or vesicle in such a manner that the target molecules are available for interaction with its intended receptor molecule which is on the surface of the intended cell. The target molecule is positioned so that it is extended away from the membrane surface. See the illustration above for a pictorial representation.

The HDV is fashioned in such a way that a connector portion is first incorporated into the membrane at the time of forming the membrane. The connector portion must have a lipophilic portion which is firmly embedded and anchored in the membrane. It must also have a hydrophilic portion which is chemically available on the aqueous surface of the vesicle. The hydrophilic portion is selected so that it will be chemically suitable to form a stable chemical bond with the target molecule which is added later. Therefore, the connector molecule must have both a lipophilic anchor and a hydrophilic reactive group suitable for reacting with the target molecule and holding the target molecule in its correct position, extended out from the vesicles' surface. In some cases it is possible to attach the target molecule to the connector molecule directly, but in most instances it is more suitable to use a third molecule to act as a chemical bridge, thus linking the connector molecule which is in the membrane with the target molecule which is extended, three dimensionally, off of the vesicle surface.

An important aspect of this invention is the fact that the target molecule can be any molecule that is recognizable by the hepatobiliary receptors of the hepatocyte. The term "hepatobiliary" is defined in the 1965 edition of Dorland's Illustrated Medical Dictionary, W.B. Saunders and Company, Philadelphia, Pennsylvania, U.S.A. as "pertaining to the liver and the bile or biliary ducts." The hepatobiliary receptors of hepatocytes are capable of recognizing and of taking into the hepatocyte a great variety of chemical structures. The nature of the target molecule is therefore most clearly defined by its biological specificity for the hepatobiliary receptors of hepatocytes.

This invention therefore can use as a target molecule, any chemical substance which can be taken into the hepatocytes and transported into the biliary system. Because of this diversity it is necessary to have this biological definition of the target molecule rather than a chemical definition.

There exists a variety of chemical structures which are chemically diverse but are generally recognized as hepatobiliary chemicals and act as effective hepatocyte target molecules. Because of the variety of chemicals that can be used by this invention as target molecules, it is necessary to provide an appropriate variety of chemical means to attach these molecules to the surface of the vesicle. This variety therefore requires a number of different connectors and chemical bridge systems to permit the use of this variety of chemical structures commonly referred to as hepatobiliary materials.

While the majority of hepatobiliary target materials will require the techniques of attachment generally described above, some will be suitable for use as a single molecule because they will have a chemical portion that acts as "connectors" and other portions that act as "target molecules." Examples of useable systems will therefore include representatives of both the multiple step (connector - bridge - target) system, and the single step system just described.

The structural formula below is an example of the result of a multiple step method of constructing a Hepatocyte Directed Vesicle (referred to as HDV):

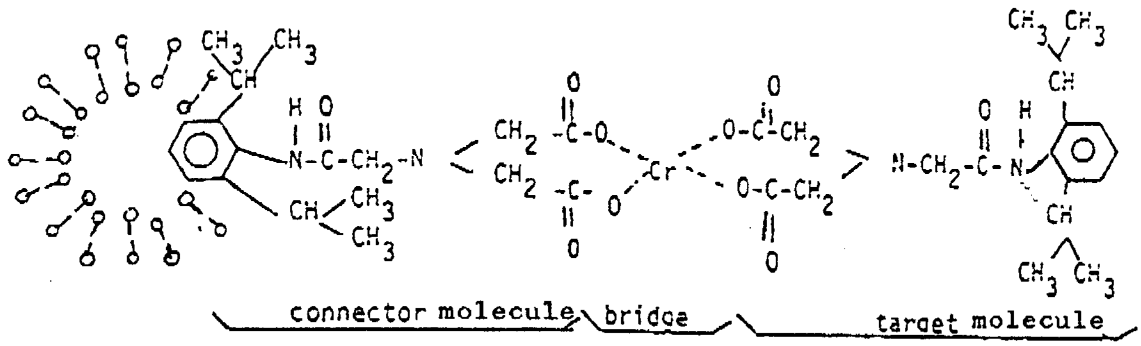

In the structure above a series of lines and small circles symbolize the positioning of bipolar lipid membranes which encircle a vesicle core volume. This structural representation is grossly out of proportion for convenience of illustration. The bipolar vesicle is actually huge in comparison to the connector-target molecule.

The bracket labelled "connector molecule" means a iminodicarboxylic acid molecule having a lipophilic and a hydrophilic end. The lipophilic end is the end containing the benzene ring and is shown embedded into the bipolar film of the vesicle. The hydrophilic portion of the connector molecule will extend away from the face of the vesicle because it is interacting with water.

The bracket labeled "target molecule" indicates in this particular illustration, an identical iminodicarboxylic acid as that in the connector molecule. This is one acceptable, and preferred embodiment, but by no means is it an exclusive requirement. That is, the connector and target molecules are illustrated as being identical in this embodiment, but that is not a requirement. It is a requirement of this preferred embodiment that the target molecule be a molecule which is recognized by the hepatobiliary receptors of the liver as opposed to sugar-type molecules which are normally attracted to the Ashwell receptors.

The bracket labeled "bridge" designates a chromium ion bridge which will connect the hydrophilic charged terminal ends of the two iminodicarboxylic acid groups. These groups would not normally connect to one another; therefore, the chromium ion is used for that purpose. There are other possibilities wherein a hepatobiliary target molecule may be directly connected to a connector molecule.

The hepatobiliary targeting molecule may be employed to bring a vesicle into that portion of the liver which normally is concerned only with creating bile fluids. After this discovery, a wide range of possible combinations of connectors and target molecules may be envisaged. It is therefore only up to the innovative chemist to select from the great number of possible combinations to achieve the necessary results.

It is not obvious that the hepatobiliary receptors will accept a system which includes a vesicle and drop off the vesicle in the hepatocyte where it releases its pharmacologic cargo and is effective, with the remainder of the system passing onto the bile duct just as would be expected of anything taken into the hepatobiliary receptors.

In the study and experiments done to validate this invention, it appears that a much larger and greater number of vesicles may be taken into the hepatocytes through the hepatobiliary receptors.

I. Chemical Compositions:

A. Preferred bulk bipolar lipid constituents (75-95 %)
   1. Distearoyl lecithin (DSL)
   2. Dipalmatoyl lecithin (DPL)
   3. Other lecithins with chain lengths C10-C20

B. Minor constituents (0.1-25 %) for stability
   1. Cholesterol
   2. Dicetyl phosphate
   3. Albumin

C. Target molecules (0.1-10%)

Hepatocyte specificity is conveyed by molecules having the following structure which have both hydrophilic and hydrophobic portions:

Preferred Target Structure 1:

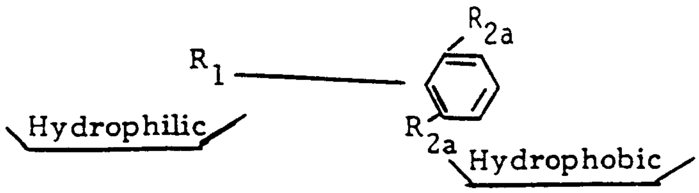


This portion is responsible for targeting

Preferred Target Structure 2:

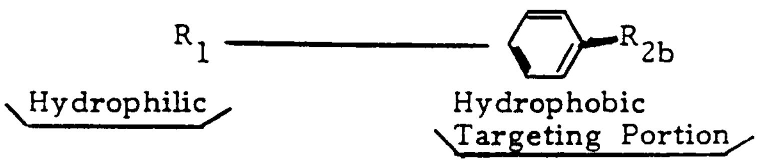


where $R_1$ has the following structure(s) and n = 1 - 3;

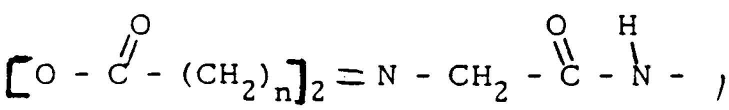

$R_{2a}$ has the following structure(s):
(1) $CH_3$, (2) $CH_2CH_3$, and (3) $CH(CH_3)_2$, and
$R_{2b}$ has the following structure:

$CH(CH_3)_2$ .

Preferred Target Structure 3:

N-(3 Bromo-2,4,6-trimethylphenylcarbamoylmethyl)-iminodiacetic acid.

Preferred Target Structure 4:

N-(3-Cyano-4,5-dimethyl-2-pyrrylcarbamoylmethyl) iminodiacetic acid.

Preferred Target Structure 5:

Biliverdin (or Bilirubin)

D. Preferred Bridge Examples

1. Inorganic salts of:
   a. Chromium
   b. Cobalt
   c. Iron
   d. Zinc
2. Organic compounds:
   a. Ethylene Diamine
   b. Propylene Diamine

Connectors

With each of the preferred target structures, the preferred connector is the same as the target. The preference is only for the convenience of the manufacturer.

It should be noted that in the target structures the diacetic acid portion provides oxygen bonds for connecting through a bridge to a connector molecule presenting similar oxygen boding points. The bridge molecules provide the necessary ligands to connect to each of the four oxygens and thereby interconnect the target and connector molecules.

Although the foregoing is preferred, it is not essential that identical molecules be used for connector and target with a bridge interconnection. An example of alternate connector means not using a bridge is to incorporate albumin into the vesicle membrane, and then react the propionyl group of bilirubin as a target with a lysine amino group to form a Schiff base. In this example, no bridge molecule is used per se.

Therefore, it is within the scope of this invention to form a single-step target system without separate connector and bridge molecules.

These examples are of materials that are incorporated into the HDV in one step, not requiring the multiple step addition of bridge and then target molecule, although portions of the large molecular conjugates can be designated as "connector", "bridge" and "target".

There are four parts forming the completed preferred HDV:

1. Vesicle, which carries the cargo
2. Vesicle connector molecule
3. Bridging molecule
4. Target molecule .

In this invention the connector molecules and the target molecules can be identical. That is to say that two identical molecules which are connected by a specific molecular bridge, when appropriately attached to the vesicle surface, form the completed HDV system.

However, it is also possible to connect two dissimilar molecules by a bridge, thereby enabling selection of various useful combinations of target and connector molecules which would not otherwise be compatible. This is within the skill of the bio-chemist after the concepts of the invention have been understood.

In the preferred target structures above for the hepatobiliary receptors where $R_1$ has the preferred structure of $n = 1$ in order to maximize the negative charge on the carboxyls, "n" may be extended with additional methylenic ($CH_2$) groups with concomitant reduction of the negative charge on the carboxyl group, which would result in a progressively weaker ligand connecting the bridge.

Preparation of a Preferred Embodiment

This development describes the coupling of a liver targeting agent to a vesicle membrane for the purpose of creating a new targeting system. There is an orderly sequence of events that is required for the successful coupling of the targeting agent to the vesicle membrane. The preparation of the vesicle targeting system initially requires the formation of a vesicle structure with a connector molecule embedded into the membrane. In the course of this development there have been two special ligands utilized to attach the connector molecules to the targeting molecules. Initially, an inorganic substance, chromium chloride, was used. Concern about long-term chromium toxicity arising from continual vesicle dosing has been dispelled by further study, but organic ligands such as $H_2N\text{-}CH_2\text{-}(CH_2)_n\text{-}NH_2$ where $n = 1\text{-}4$, (i.e. for example ethylene diamine) is an alternative for coupling purposes. However, from a practical point of view, both ligands serve the same function.

Described below is a reaction sequence relating to the various steps involved in the formation of an HDV system. Each step is unique in relation to the next step and each subsequent step in the reaction sequence. This is the preferred embodiment.

The first step of the sequence requires that the connector molecule and the lipid components comprising L--distearoyl lecithin (DSL), dicetyl phosphate (DCP) and cholesterol (CHOL) in conjunction with human serum albumin (HSA) be formulated into a vesicle structure with a bipolar membrane.

As a result, the connector molecule is oriented in three-dimensional space as noted above. This three-dimensional position of the connector molecule creates the foundation for the successive reactions resulting in the structures completing the HDV. At this step the entire vesicle is acting as a large suspended structure with the vesicle surface interspersed by a uniform distribution of connector molecules, three-dimensionally poised to partake in the next reaction sequence. The hydrophilic portion of the connector molecule projects into the aqueous phase of the media, whereas the hydrophobic portion of the molecule is buried in the lipophilic section of the membrane. Since the molecular size of the connector molecule is small in

comparison to that of a vesicle and since only a portion of the connector molecule is entrapped in the vesicle structure at the time of preparation, the remaining unentrapped connector molecules can be removed easily by Sephadex G-100-120 gel filtration chromatography.

In the next step a five-fold molar excess with respect to the initial concentration of connector molecules, of either $CrCl_3$ hexahydrate or ethylene diamine (which are examples of bridging molecules), is reacted with the vesicle structure containing the embedded connector molecule to form a vesicle connector molecule-bridging molecule complex. As a result of this step, the proper three-dimensional orientation of the bridge molecule is established for the subsequent binding of a targeting molecule to the vesicle-connector molecule-bridging molecule complex.

In the final step the target molecules are added to the vesicle-connector-bridge complex. A five-fold molar excess of target molecules is reacted with the vesicle structure containing the vesicle connector molecule-bridging complex to form the vesicle connector molecule-bridging molecule-target molecule conjugate to be used for vesicle targeting purposes. The excess unreacted target molecule may then be removed by Sephadex G-100-120 gel filtration chromatography.

In summary, the unique steric symmetry and structure of the targeting molecule permits it to be functional and useful in its targeting role. This unique, three-dimensional symmetry can be achieved through utilizing the reaction sequences as described in the aforementioned paragraphs.

The new chemistry featured in this targeting system is that a molecule that is both hydrophilic and hydrophobic can be converted to a hydrophobic targeting moiety with the concomitant neutralization of the charged portion of the molecule through ligand formation and, in addition, provide the correct three-dimensional orientation or projection of its hydrophobic targeting portion into the hydrophilic aqueous media.

Thus, the sequence in which these molecules are reacted promotes and establishes the proper orientation of the insoluble hydrophobic group in spite of the 'unfriendly' environment posed by the aqueous media toward hydrocarbon structures.

Furthermore, the negative charge contributed by dicetyl phosphate (one of the membrane constituents) at the surface of the vesicle, creates charge-charge repulsion between vesicles and facilitates their suspension and shelf-life stability. The negatively charged phosphate at the hydrophilic end of the molecule at neutral pH is responsible for the charge-charge repulsion effect resulting in vesicle stabilization.

This charge-charge repulsion is strong enough (probably by two-orders of magnitude) to overcome any van der Waals induced dipole interactions caused by the hydrophobic phenyl ring and the accompanying R-groups.

Method for Preparing HDV

DSL 69.12 mg; iminodiacetic acid complex 1.07 mg; DCP 14.1 mg; CHOL 5.0 mg .

1.5 ml of $CHCl_3$/MeOH (2:1 v/v) was added to solubilize the reagents. The sample was placed on the rotoevaporator and taken to dryness at 60 ± 0.5 °C with slow turning under aspirator vacuum. Then 2.4 ml of freshly prepared 40mM phosphate buffer pH 7.4 with a concentration of HSA and serotonin, the active agent at 4.2 mg/ml and 10 mg/ml, respectively, were added to the dried lipid components. The sample was sonicated (using a Heat Systems Ultrasonic Cell Disruptor at setting #4 equipped with a transducer and cup horn) at 60 ± 0.5 °C for 15 minutes. Next, the sample was annealed at 60 ± 0.5 °C with slow turning for 15 minutes. Then the sample was centrifuged (in a Triac Clinical Centrifuge) at the blood setting for 15 minutes at room temperature.

Then, 1.5 ml of the supernatant was chromatographed on a 1.5 x 25.0 cm Sephadex G-100-120 column that had been previously equilibrated with 40mM phosphate buffer pH 7.4. This first chromatography was performed in order to remove the unentrapped HSA and serotonin, the active agent. The lipid vesicles were collected and then, with respect to the initial concentration of vesicle connector molecules, were reacted with a five-fold molar excess of $CrCl_3$. The vesicles were then rechromatographed using the same buffer to remove unreacted $CrCl_3$. The collected vesicles were then reacted with a five-fold molar excess of connector molecules. Following this step the vesicles were then rechromatographed using the same buffer system to remove unreacted connector molecules. Following this final chromatography, the vesicles were stored under nitrogen in the refrigerator at 5 °C. Phenyl-mercuric nitrate may be used as a preservative (0.001 %).

In Vivo Testing of the Preferred Embodiment

To test the invention an in vivo model is used. It must be born in mind that the test is for successful delivery of a pharmaceutical dose to the liver. The delivery of the cargo is established by demonstrating the desired pharmacological response to the cargo by the liver.

The entire thrust of this invention, hence the disclosure of the many preferred approach methods, is to establish factually by in vivo testing that the pharmaceutical load carried within a vesicle can and is delivered to the liver of a warm-blooded animal and that the pharmacological cargo is made available to act at the hepatocyte. In order for the pharmacological cargo to act on its receptor, the HDV must be effectively dismantled, releasing the cargo from the protective coating of the vesicle. The pharmacological cargo may then be used by the liver to cause a hormonal control of the liver as in the natural functioning state of healthy individuals.

It has also been discovered and established beyond doubt that the liver storage of glucose eaten during a meal requires not only the hormone insulin, but also that a co-factor is required for proper liver function. That co-factor is serotonin. Serotonin is a chemical, 5-hydroxytryptamine (5-HT), present in platelets, gastrointestinal mucosa, mast cells, and in carcinoid tumors. Serotonin is a potent vasoconstrictor (Tabers' Cyclopedic Medical Dictionary, 14th Edition). It has been established that serotonin is supplied to the portal vein leading to the liver while food is being absorbed by the intestines and is controlled by the central nervous system. Thus, it was discovered that by severing the vagus nerve the serotonin in the portal vein could be essentially eliminated. When this is done, it is now established, the liver no longer will convert the nutrient glucose to glycogen and store the glycogen. Rather, the liver will allow all of the glucose to proceed into the peripheral system, thereby producing excess sugar in the blood and providing the symptoms of diabetes. Although there may be sufficient insulin available at all times at the liver, a deficiency of serotonin will result in an excess of glucose in the blood.

By providing a serotonin load in this hepatocyte delivery vesicle and targeting the vesicle to the hepatobiliary receptors of the liver, and observing the return to normal liver function, the ability of the target vesicle system of this invention to effectively deliver a pharmaceutical load to the liver, whatever the load may be, has been clearly established. If diagnostic material is desired, or insulin, or as in the case of hypoglycemia, the blocking agents for the serotonin function, the unique capability of this invention has been established.

Hence, in a dog model having a normal insulin production by the pancreas glands, it is a superior test of the HDV to denervate the glands producing serotonin, and after establishing diabetes symptoms of excess blood glucose, to direct serotonin (5HT) to the liver. Re-establishment of normal liver glucose control then proves effective HDV delivery to the hepatocytes.

In a first study testing the HDV system, a chronic dog model was selected which mimics the early stages of adult onset diabetes mellitus, now referred to as diabetes mellitus Type II. In this model, the diabetes was induced by selective denervation. The normal healthy dogs prior to denervation respond to a standard meal (one-third carbohydrate) by having slightly lowered levels of peripheral blood glucose. Following denervation, the dogs maintain normal fasting blood glucose values, but their peripheral blood glucose levels rise significantly after a standardized meal, thus responding in a similar manner to adult onset diabetes.

This particular model was selected for this study because it enabled the evaluation of the HDV system in alert, unanesthesized animals.

This study was divided into three phases, studying the blood glucose response of the animals while in the (1) normal state; (2) diabetic-like state; and (3) state of successful treatment of the diabetic-like state with subcutaneously administered HDV containing serotonin.

The experimental plan required four normal, healthy mongrel dogs. This first phase of the study was to determine the blood glucose response of these four dogs to a standardized glucose meal, comparable to the oral glucose tolerance test in people.

The obtained results are shown in the drawings.

Fig. 1 is a graph depicting the plasma glucose levels of normal dog models which were denervated, tested for diabetes and then normalized by this invention;

Fig. 2 is a bar graph summarizing the data of Fig. 1;

Fig. 3 is a graph of blood sugar level after a controlled dose of saline on a diabetic fasting dog, and compared result after administering HDV/5-HT;

Fig. 4 is a graph showing the change from glucose output to uptake following infusion of HDV insulin (HDV I);

Fig. 5 is a graph comparison of the effect of correcting the lack of control serotonin on diabetic animals via an oral dose; and

Fig. 6 is a graph comparison of the effect of correcting the lack of control serotonin on diabetic animals via intravenous injection.

The graph of Fig. 1 is a comparison chart for in vivo testing.

The data for this normal phase are shown in the graph as the line indicated by reference number 10. The data are expressed as a percentage of the fasting blood glucose value taken prior to feeding. Since four dogs were used, the average or mean value for the data is plotted. The data are statistically analyzed, and the variation in the data is shown by the small vertical bars above and below the data points. These bars are the standard error of the mean (SEM).

Following the acquisition of the data described above, the dogs were surgically denervated to induce the diabetic-like state and allowed a week to recover. At this time the study is again repeated, and the data are shown as the line indicated by reference number 12, along with the error bars (SEM). It is clearly seen from the data that the animals' responses were different following the denervation. The asterisks (*) at the 1, 2, 3 and 4 hour data point along the line 10 indicate that by statistical analysis of the data by the conventional student's t test, the blood glucose response of the dogs following surgery is statistically different from the response of the dogs prior to surgery. The level of significance is less than 5 % (designated, therefore, as $p < .05$). This means that there is less than a 5 % chance that the difference observed would have happened randomly.

The third phase of the study was to repeat the standardized meal in the diabetic-like dogs, but with the dogs injected subcutaneously with 1.0 ml of HDV containing serotonin. The total dose of serotonin in the HDV was 150 μg serotonin, and it was administered one hour prior to eating and immediately after taking the baseline blood glucose sample. The response of the HDV-serotonin-treated denervated dogs is shown in line 14. The abnormal elevation of the blood glucose has been normalized. At one, two and four hours the data points were $p < 0.06$, $p < 0.05$ and $p < 0.05$ for this treatment, compared to the post-denervation meal 12.

The data from these studies are summarized in the bar graph of Figure 2.

The first bar shows that the average value for the blood glucose (hours 1-4) for the dogs prior to surgery decreased about 10 % after a meal compared to their blood glucose value taken prior to eating. The second bar shows that the mean blood glucose response (hours 1-4) was increased after a meal, and the asterisk indicates that is was statistically significant ($p < 0.01$, student's t test). The denervation had caused an elevation in blood glucose following a meal, thus inducing a diabetes-like state. The third bar shows the effect of the HDV-serotonin treatment. The HDV serotonin significantly decreased the elevation of the blood glucose following a meal.

It is most important to know that in studies in connection with the invention it has been established that serotonin administered at this dose (150 μg) is not effective either intravenously or subcutaneously in inducing the effect seen with the HDV-serotonin in these studies.

The conclusions from these data are:

1. Denervation produced a diabetic-like state.
2. The effects of denervation could be corrected with very low doses of HDV containing 5-HT.

A second study tested the hypoglycemic (blood glucose-lowering ability) effectiveness of HDV containing 5-HT in fasting (non-fed) dogs. The graph of Figure 3 summarizes the results. Four dogs (denervated as in the first study) were used in the study on two different days. The first day tested the effect of saline and the second day, the effect of HDV (with 5-HT) on fasting plasma glucose levels. The protocol required that a baseline plasma glucose be obtained and followed by a 1.0 ml subcutaneous injection of saline (day 1) or HDV/5-HT (day 2). One hour later a second blood sample was obtained for a post-treatment plasma glucose. The post-treatment glucose was compared to the baseline plasma glucose values. The saline treatment (control) experiment resulted in an increased plasma glucose compared to its baseline values. However, the HDV/5-HT treatment resulted in a statistically significant ($p < 0.01$) reduction in fasting plasma glucose.

The conclusions based on these data are:

1. Control saline injections in fasting dogs produced a slight (statistically insignificant) increase in the fasting plasma glucose.
2. HDV/5-HT injections produced a statistically significant ($p < 0.01$ by student's t test) reduction of the fasting plasma glucose.

The second conclusion, namely the administration of the HDV/5-HT and the resultant reduction in fasting plasma glucose is quite significant. Bearing in mind that the purpose for the foregoing test is to establish conclusively that the HDV delivers pharmacologic agents to the liver. Therefore, this invention,

having first established the function in serotonin in programming the liver to uptake glucose, and then administering the serotonin in the HDV with the resultant reduction in glucose, establishes beyond any reasonable doubt that the HDV has been taken into the liver and has caused the function expected of serotonin of ceasing glucose output and beginning glucose uptake. Following the initial in vivo testing of four dogs as listed above, additional tests on animals have been undertaken using four different programs. These four separate programs are outlined below and then summarized.

Hereafter is a description of four supplemental experiments which document the versatility of the HDV system. In the original tests above, experimental data for the hepatocyte delivery of serotonin are included. This system utilized the connector, bridge and target molecule complex. That original study utilized the subcutaneous route of administration. The features of the original experiment and the four supplement experiments are summarized in Table I. The important variables are:

| SUPPLEMENTAL EXPERIMENTS | | | | | |
|---|---|---|---|---|---|
| | Experiment described in original experiments | 1 | 2 | 3 | 4 |
| Connector | 2,6-diisopropylphenylcarbamoylmethyliminodiacetic acid | same | same | same | same |
| Bridge | Chromium | Chromium | Chromium | Chromium | Chromium |
| Target molecule | 2,6 diisopropylphenyl carbamoylmethyliminodiacetic acid | same | same | same | Biliverdin |
| Hormone | Serotonin | Insulin | Growth hormone | Serotonin | Serotonin |
| Route of Administration | Subcutaneous | Intravenous | Subcutaneous | Intraduodenal | Intravenous |
| Fraction of dose of horhormone for effect | 1/100th | 1/200th | 1/7th to 1/50th | 1/300th | 1/100th |

1. Target molecule. The original experiment and the first three supplemental experiments used the target molecule disclosed hereinabove. Supplemental experiment 4 used biliverdin. The differences between these two materials are very significant. The first, N-(2,6-diisopropylphenylcarbamoylmethyl)-imino diacetic acid is a synthetic material. Biliverdin is a naturally-occurring metabolite in the body that is used to form bile. Since serotonin HDV worked with both target molecules, it is established that the hepatobiliary receptor is an effective target mechanism. There is no known reference in the medical literature where a naturally-occuring metabolite, such a biliverdin, has been shown to carry and then render effective a pharmacologic cargo.

2. Different hormones. The original experiment used serotonin as its pharmacological cargo. The liver's response was a conversion of hepatic glucose output to uptake in selectively denervated animals. The supplemental experiments use growth hormone and insulin, as well as serotonin. All three hormones were efficacious when delivered by HDV. The serotonin was effective with both target molecules. It is also very important to recognize the superpotency conveyed to the hormones by the HDV system. Only a small fraction of the usual dose of the hormone that is required for a response is necessary for the HDV system.

3. Different routes of administration. The HDV system is capable of delivering drugs and hormones by the three major routes of administration: Subcutaneous injection, intravenous (or arterial) infusion, and intraduodenal (oral). In the matter of the intraduodenal administration, it should be understood that the important feat to be accomplished is the transfer of the HDV from intestinal lumen through the intestinal tissues into the blood. This has been accomplished as evidence by the pharmacological response. The further development of the HDV to an oral dose form is expected to be rather routine by those experienced in the art and science of dose formulations.

As a result of these data, it has been experimentally shown that the hepatobiliary receptor is used to target the HDV and that molecules, synthetic or natural metabolites, which appear in some form in the bile, are suitable target molecules for this invention. Also, HDV is not selective for the carrying of one hormone, but it is a generic carrier of all drugs and hormones to hepatocytes. Furthermore, the HDV is a delivery system usable for the administering of drugs and hormones via the gastrointestinal tract (oral or rectal), and the HDV can also be used parenterally (subcutaneously, intravenously, intra-arterially, etc.).

Heretofore, in this disclosure, the connector or bridge has not been varied. The critical variables are: 1) broad specificity of target molecules; 2) the ability of HDV to be administered both oral and parenterally; 3) the ability of HDV to carry different kinds of hormones; and 4) the superpotency of the drugs and hormones. Now that these critical points are experimentally demonstrated, the connector and bridge may be varied. However, the target molecule needs to be attached to the surface of the vesicle. Once the chemical natures of the vesicle membrane and the target molecule are known it is possible to construct a multitude of workable connectors and bridges. The main requirements for connectors are to be lipophilic and insertable into the membrane and to have a compatible reactive group, such as

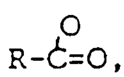, or 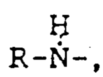, or R-S-,

which can be coupled either directly to the target molecule or indirectly to the target molecule by a bridge molecule. Examples of bridges are given above.

SUPPLEMENTAL EXPERIMENT 1

HDV Insulin (HDV-I): Intravenous

| | |
|---|---|
| Connector: | N-(2,6-diisopropylphenylcarbamoylmethyl)-iminodiacetic acid |
| Bridge: | Chromium |
| Target: | N-(2,6-diisopropylphenylcarbamoylmethyl)-iminodiacetic acid |
| Hormone: | Insulin |
| Route of administration: | Intravenous |

The purpose of this experiment was to demonstrate the efficacy of HDV insulin, constructed with the above materials, in an insulin-deficient (diabetic) dog. The response of HDV insulin to be measured was the conversion of hepatic glucose output to uptake by measuring the glucose (Beckman Glucose Analyzer)

levels of simultaneously obtained portal and hepatic vein blood. Diabetes had been induced by the method of Black (Am. J. Pathol. 98, No. 2 (1980) 295 - 305), and sampling was done via portal and hepatic vein catheters acutely placed under general anesthesia. The dog had been fasted from food for twenty-four hours and taken off of exogenously-administered insulin for forty-eight hours.

The state of hepatic glucose output occurs when the hepatic vein glucose level (in mg-%) exceeds the portal vein glucose level. Hepatic glucose uptake is the reverse.

The data for the experiment are found in the graph of Figure 4. At "0" minutes the dog is in glucose output, and this output is maintained through the "23" and "35" minute samples, even though glucose is infused via a mesenteric vein at a rate of 0.5 g/kg body weight/hour. An intact (non-diabetic animal) would have immediately converted to glucose uptake. This dog's diabetic state is confirmed by its' not converting to glucose uptake at these times.

Following the "35" minutes sample, HDV insulin was infused via the left external jugular vein at a rate of 0.03 mU/kgmin. The dog's conversation to hepatic glucose uptake is dramatically seen at 48, 55, 68 and 80 min, even though the HDV-I infusion ceased at 60 min. The minimum infusion rate for insulin alone is 6.25 mU/kgmin. Thus, this effective dose of HDV-I was 1/200th of the regular insulin dose, documenting the super-potency of the HDV-I in a diabetic dog by intravenous infusion.

SUPPLEMENTAL EXPERIMENT 2

HDV Growth Hormone (HDV-GH) : Subcutaneous

| | |
|---|---|
| Connector: | N-(2,6-diisopropylphenylcarbamoylmethyl)-iminodiacetic acid |
| Bridge: | Chromium |
| Target: | N-(2,6-diisopropylphenylcarbamoylmethyl)-iminodiacetic acid |
| Hormone: | Growth Hormone |
| Route of administration: | Subcutaneous |

The purpose of this experiment was to demonstrate the efficacy of HDV-GH, constructed with the above materials, in stimulating growth in hypophysectomized rats. The HDV-GH was administered to rats daily by subcutaneous injection. The control rats required 10 µg GH per day (also given subcutaneously) to maintain normal growth of 1 g weight gain per day. The HDV-GH treated rats attained normal growth rates with dosages of HDV-GH of 0.15 to 1.5 µg per day. Thus, the HDV-GH required only 1/7th to 1/50th of the regular dose of growth hormone. The HDV-GH was superpotent and was efficacious when given by subcutaneous injection.

SUPPLEMENTAL EXPERIMENT 3

HDV Serotonin (HDV-S): intraduodenal (Oral)

| | |
|---|---|
| Connector: | N-(2,6-diisopropylphenylcarbamoylmethyl)-iminodiacetic acid |
| Bridge: | Chromium |
| Target: | N-(2,6-diisopropylphenylcarbamoylmethyl)-iminodiacetic acid |
| Hormone: | Serotonin |
| Route of administration: | Intraduodenal injection (oral) |

The purpose of this experiment was to demonstrate the efficacy of HDV serotonin, constructed with the above materials, in a selectively denervated dog that was not insulin-deficient, but in a diabetic-like state. In this model, the response of the HDV serotonin was the conversion of hepatic glucose output to uptake. This conversion was determined by measuring the glucose levels (Beckman Glucose Analyzer) in simultaneously obtained blood samples from the portal and hepatic veins. Sampling was by means of sampling catheters acutely placed under general anesthesia.

The state of hepatic glucose output occurs when the level of glucose (mg-%) in the hepatic vein exceeds that of the portal vein. Hepatic glucose uptake is the reverse situation.

Data for the experiment are found in the graph of Figure 5. At "0" minutes, during the control period, the animal is in a state of hepatic glucose output. Because of the selective denervation, the animal stays in output (at 20 and 55 min), even though glucose is being infused at a rate of 0.5 g/kg body weight/hour via a mesenteric vein.

The HDV serotonin is injected into the duodenum (0.8 ml containing 3 µg serotonin/kg) at 60 min. At 80 and 100 min, the HDV Serotonin has converted the hepatic glucose output to uptake.

The conclusions are: 1) HDV serotonin can cross from the lumen of the intestine into the blood and deliver HDV serotonin to the hepatocyte where the serotonin is released and can carry out its pharmacologic function, and 2) HDV serotonin is superpotent. The single intraduodenal injection of serotonin (3 µg/kg) produced a pharmacologic effect that would have required an intraportal infusion of 10-30 µg/kg body weight/min for thirty minutes (total infused dose of serotonin per kg body weight). The intraduodenal dose was 1/300th of the necessary intraportal dose of free hormone.

SUPPLEMENTAL EXPERIMENT 4

HDV Serotonin : Intravenous

| | |
|---|---|
| Connector: | N-(2,6-diisopropylphenylcarbamoylmethyl)-iminodiacetic acid |
| Bridge: | Chromium |
| Target: | Biliverdin |
| Hormone: | Serotonin |
| Route of administration: | Intravenous |

The purpose of this experiment was to demonstrate the efficacy of HDV Serotonin, constructed with the above materials, in a selectively denervated dog. This dog, when infused with glucose, has portal vein glucose values (mg-%, measured with a Beckman Glucose Analyzer) that are less than hepatic vein glucose, indicating a net hepatic glucose output. This is clearly seen in the data presented in the graph of Figure 6. The dog is in a state of glucose output for time 15, 30 and 40 min. The HDV serotonin was then infused at a rate of 0.3 µg/kg body weight/minute, along with the glucose. The hepatic glucose output promptly converted to uptake and was maintained even 30 minutes after discontinuing the HDV Serotonin infusion.

This experiment demonstrates the efficacy of a different hepatobiliary target molecule, biliverdin. Its structure is markedly different from the N-(2,6-diisopropylphenylcarbamoylmethyl)-imino diacetic acid used in the other experiments. The experiment also shows the typical HDV superpotency. In this experiment the HDV serotonin was infused at a rate of 0.3 µg/kg/min. The rate of free serotonin infusion required is 30 µg/kg/min. The superpotency in this case is 100-fold.

The following appendix sets out a class of hepatobiliary imaging agents which has been compiled to illustrate many possible targeting agents within the scope of this invention. These have all been compiled using the guide criteria that the target be a recognized hepatobiliary agent, and that a person of ordinary skill as a bio-chemist would be able to follow the foregoing teaching to use these materials successfully as a hepatobiliary target for a lipid vesicle.

APPENDIX

Substituted Iminodiacetate (IDA) Complexes

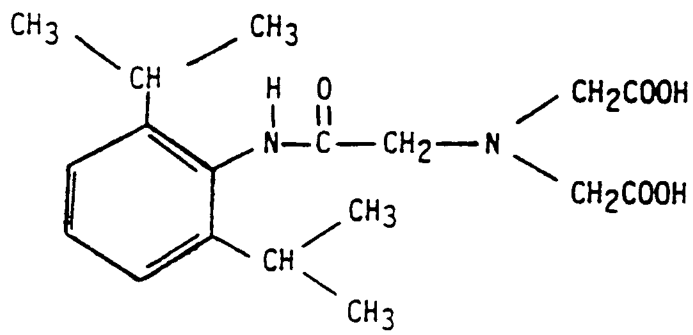

N-(2,6 - diisopropylphenylcarbamoylmethyl)-iminodiacetic acid (DISIDA) (Hepatolite)

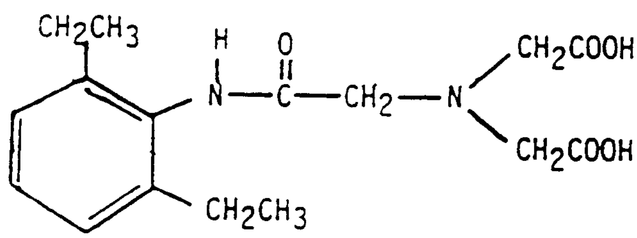

N-(2,6 - diethylphenylcarbamoylmethyl)-iminodiacetic acid (DIDA)

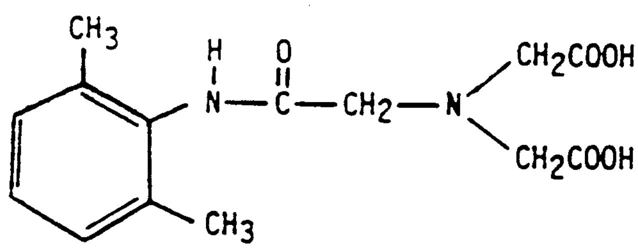

N-(2,6 - dimethylphenylcarbamoylmethyl)-iminodiacetic acid (HIDA)

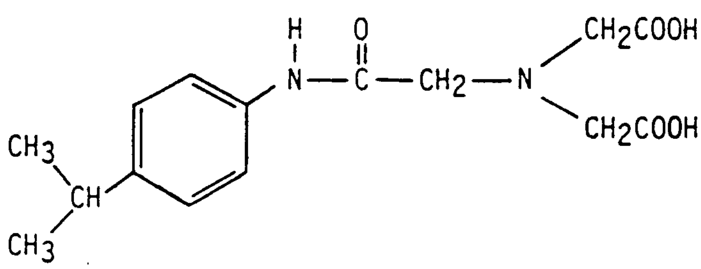

N-(4-isopropylphenylcarbamoylmethyl)-iminodiacetic acid (PIPIDA)

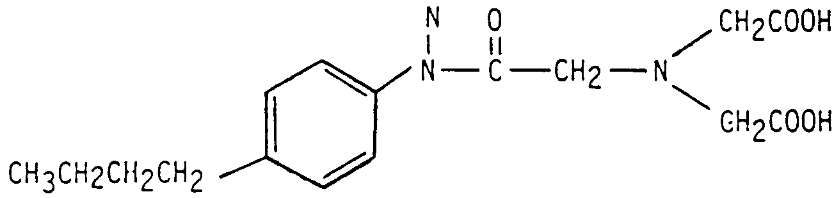

N-(4-butylphenylcarbamoylmethyl)-iminodiacetic acid (BIDA)

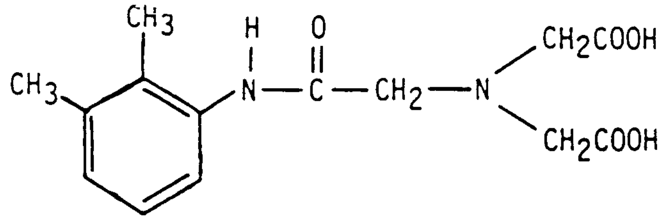

N-(2,3 - dimethylphenylcarbamoylmethyl)-iminodiacetic acid

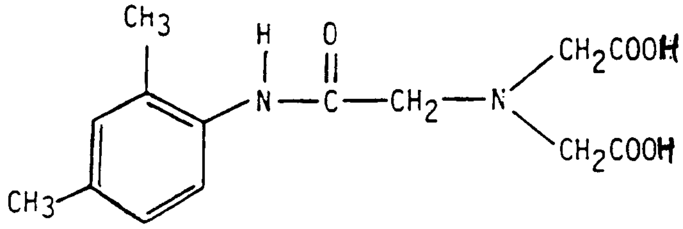

N-(2,4-dimethylphenylcarbamoylmethyl)-iminodiacetic acid

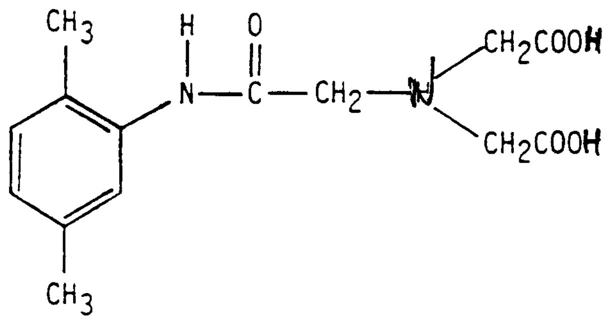

N-(2,5-dimethylphenylcarbamoylmethyl)-iminodiacetic acid

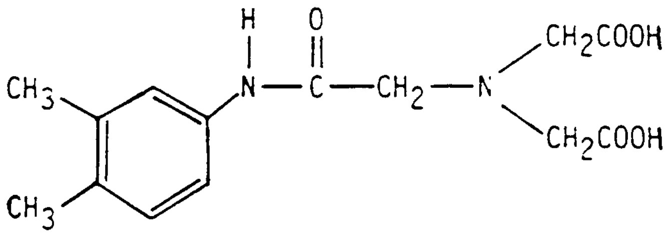

N-(3,4-dimethylphenylcarbamoylmethyl)-iminodiacetic acid

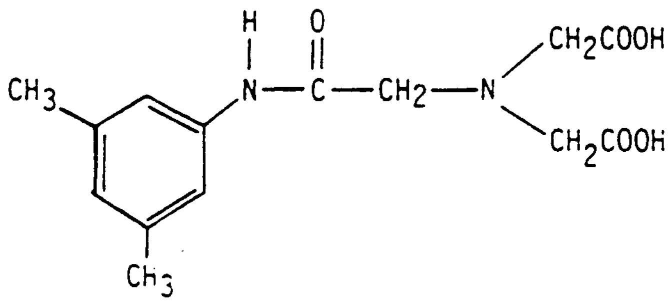

N-(3,5-dimethylphenylcarbamoylmethyl)-iminodiacetic acid
N-(3-butylphenylcarbamoylmethyl)-iminodiacetic acid
(meta butyl)
N-(2-butylphenylcarbamoylmethyl)-iminodiacetic acid
(ortho butyl)
N-(4-t-butylphenylcarbamoylmethyl)-iminodiactic acid
(para tertiary butyl)
N-(3-butoxyphenylcarbamoylmethyl)-iminodiacetic acid
(meta butoxy)
N-(2-hexyloxyphenylcarbamoylmethyl)-iminodiacetic acid
(ortho hexyloxy)
N-(4-hexyloxyphenylcarbamoylmethyl)-iminodiactec acid
(para hexyloxy)
azo substituted iminodiacetic acids
iminodicarboxymethyl-2-naphthyl ketone
phthalein, complexone
N-(5-pregnene-3-$\beta$-ol-2-oylcarbamoylmethyl)-iminodiacetic acid
3$\alpha$, 7$\alpha$, 12$\alpha$-trihydroxy-24-norcholanyl-23-iminodiacetic acid
N-(3-bromo-2, 4, 6-trimethylphenylcarbamoylmethyl)-iminodiacetic acid
benzimidazol methyl iminodiacetic acid

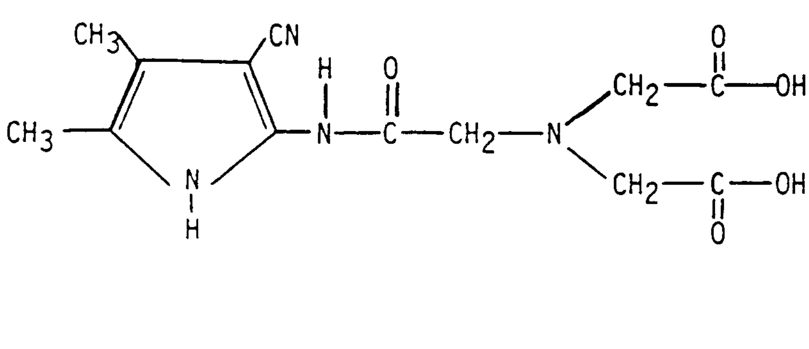

N - (3-cyano-4,5-dimethyl-2-pyrrylcarbamoylmethyl)-iminodiacetic acid

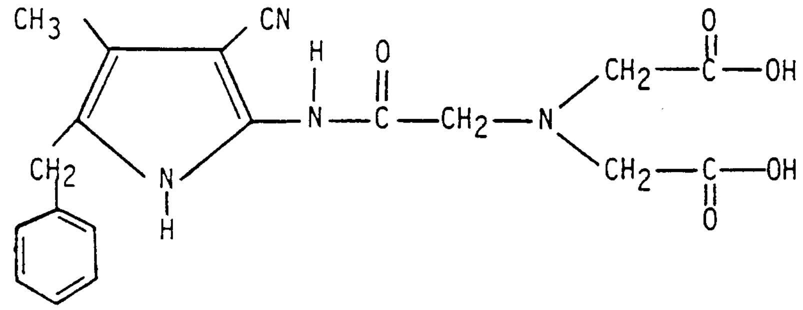

N - (3-cyano-4-methyl-5-benzyl-2-pyrrylcarbamoylmethyl)iminodiacetic acid Other Derivatives of N - (3-cyano-4-methyl-2-pyrrylcarbamoylmethyl)-iminodiacetic acid:

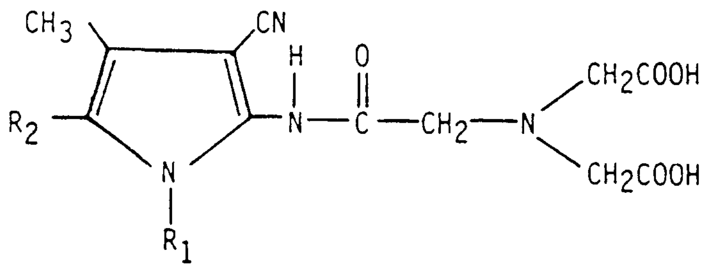

| $R_1$ | $R_2$ |
|---|---|
| H | iso - $C_4H_9$ |
| H | $CH_2CH_2SCH_3$ |
| H | $CH_2C_6H_4$-$p$-OH |
| $CH_3$ | $CH_3$ |
| $CH_3$ | iso - $C_4H_9$ |
| $CH_3$ | $CH_2CH_2SCH_3$ |
| $CH_3$ | $C_6H_5$ |
| $CH_3$ | $CH_2C_6H_5$ |
| $CH_3$ | $CH_2C_6H_4$-$p$-$OCH_3$ |

ethylenediamine - N, N - bis(2-hydroxy-5-bromophenyl)-acetate
N'-acyl- and N'-sulfonylethylenediamine - N, N-diacetic acid
N'-substituted derivatives of ethylenediamine - N, N-diacetic acid (EDDA):

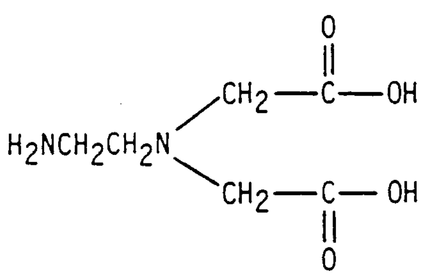

N'-acetyl EDDA

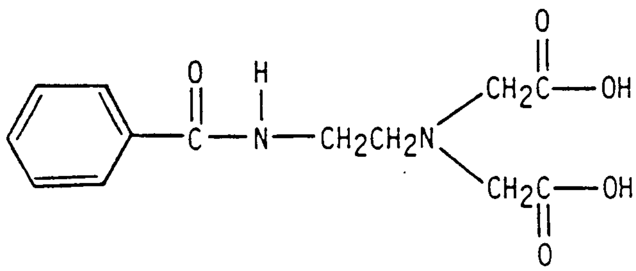

N'-benzoyl EDDA

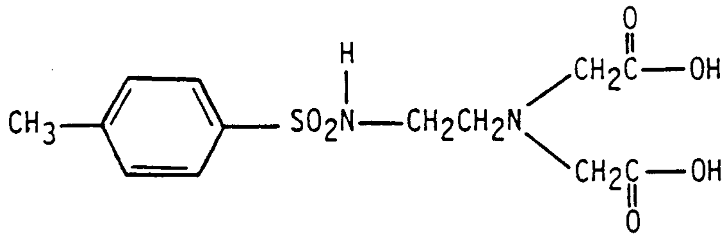

N'-(p-toluenesulfonyl) EDDA

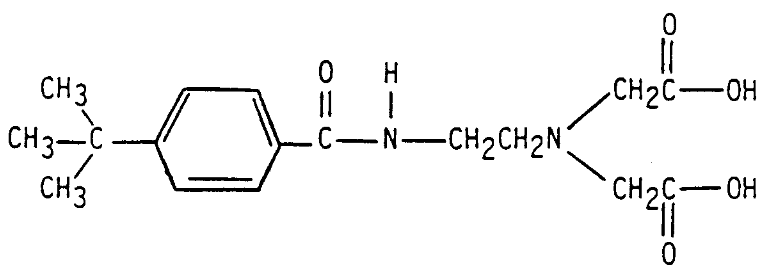

N' - (p-t-butylbenzoyl) EDDA

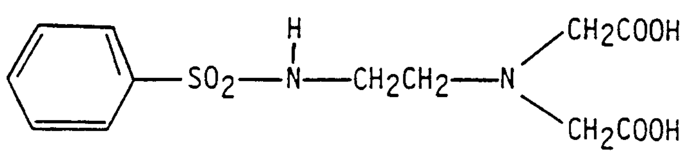

N'-(benzenesulfonyl) EDDA

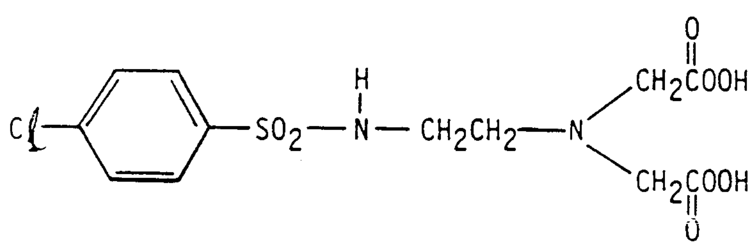

N'-(p-chlorobenzenesulfonyl) EDDA

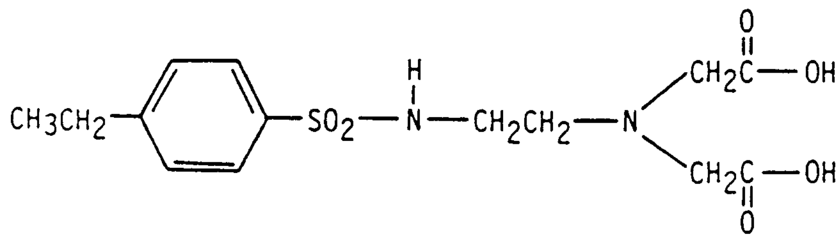

N' - (p-ethylbenzenesulfonyl) EDDA

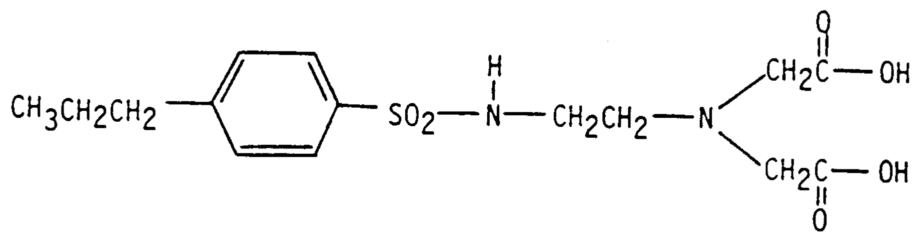

N' - (p-n-propylbenzenesulfonyl) EDDA
N' - (naphthalene-2-sulfonyl) EDDA

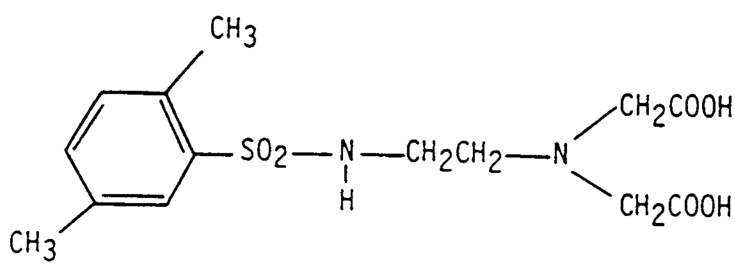

N' - (2, 5 - dimethylbenzenesulfonyl) EDDA

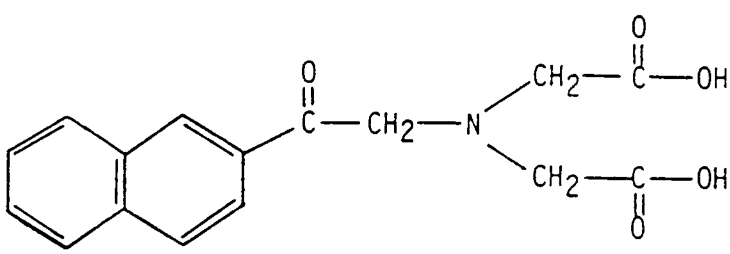

N - (2-acetylnaphthyl) - iminodiacetic acid (NAIDA)

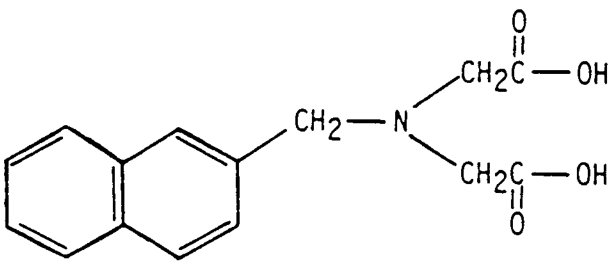

N - (2-naphthylmethyl) - iminodiacetic acid (NMIDA)

Hepatobiliary Dyes

rose bengal
congo red
bromosulphthalein
bromophenol blue
phenolphthalein
toluidine blue
indocyanine green

Hepatobiliary Contrast Agents

iodipamide
ioglycamic acid (Biligram)

Bile Salts

bilirubin
cholyglycyliodohistamine
thyroxineglucuronide

Hepatobiliary Thiol Complexes

penicillamine
β-mercaptoisobutyric acid
dihydrothioctic acid
6-mercaptopurine
kethoxal-bis(thiosemicarbazone)

Hepatobiliary Amine Complexes

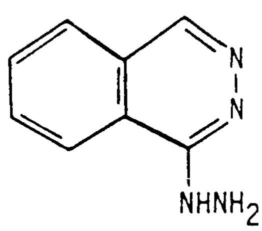

1 - hydrazinophthalazine (hydralazine) sulfonyl urea

Hepatobiliary Amino Acid Schiff Base Complexes

pyridoxylidene glutamate
pyridoxylidene isoleucine
pyridoxylidene phenylalanine
pyridoxylidene tryptophan
pyridoxylidene 5-methyltryptophan
additional pyridoxylidene aminates
3-hydroxy-4-formyl-pyridene glutamic acid

Miscellaneous Hepatobiliary Complexes

tetracycline

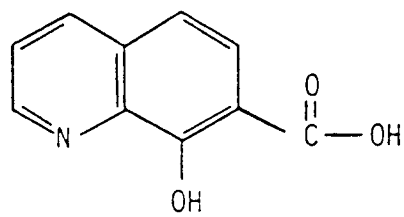

7-carboxy-8-hydroxyquinoline
phenolphthalexon
eosin
verograffin

## Claims

1. Compositions consisting of or comprising hepatocyte-directed vesicles consisting of a bipolar lipid membrane enclosing one or more biological, pharmaceutical or diagnostic chemicals and having attached to the lipid membrane targeting molecules extending away from the vesicle, characterized in that the targeting molecules have biological specifity for the hepatobiliary receptors of hepatocytes.

2. The compositions according to claim 1, characterized in that the targeting molecule comprises a lipophilic end attached to the membrane structure and a targeting portion having biological specifity for the hepatobiliary receptors of hepatocytes.

3. The compositions according to claim 2, characterized in that the targeting molecule has a targeting portion of the formula

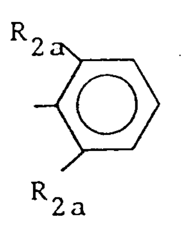

wherein $R_{2a}$ is $CH_3$, $CH_3$-$CH_2$ or $CH(CH_3)_2$.

4. The compositions according to claim 2, characterized in that the targeting molecule has a targeting portion of the formula

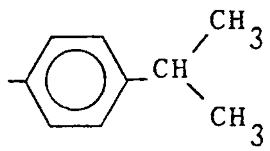

.

5. The compositions according to claims 1 to 4, characterized in that the targeting molecule is a substituted iminodiacetate complex.

6. The compositions according to claim 2, characterized in that the targeting molecule has a targeting portion which is N-(3-bromo-2,4,6-trimethylphenylcarbamoylmethyl)-imino diacetic acid, N-(3-cyano-4,5-dimethyl-2-pyrrylcarbamoylmethyl)-imino diacetic acid, bilirubin or biliverdin.

7. The compositions according to claims 2 to 6, characterized in that the targeting molecule is composed of a connector molecule having a lipophilic end attached to the membrane structure and a target molecule comprising the targeting portion, the connector molecule and the target molecule being linked together either directly or by means of a bridge.

8. The compositions according to claim 7, characterized in that the connector molecule and the target molecule comprise hydrophilic charged terminal ends linked together by means of a chromium, cobalt, iron or zinc ion.

9. The compositions according to claim 7, characterized in that the connector molecule and the target molecule are linked together by means of a bridge of the formula

$H_2N-CH_2-(CH_2)_n-NH_2$,

wherein n is 1-4.

10. The compositions according to claim 7, characterized in that the target molecule has the formula

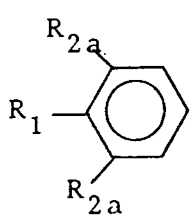

or the formula

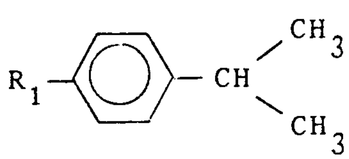

wherein

$R_1$ is $[HOOC-(CH_2)_n-]_2=N-CH_2-CO-NH-$n being 1-3, and

$R_{2a}$ is $CH_3$, $CH_3-CH_2$ or $CH(CH_3)_2$.

11. The compositions according to claims 1 to 3 and 5 to 8, characterized in that the targeting molecule has the formula

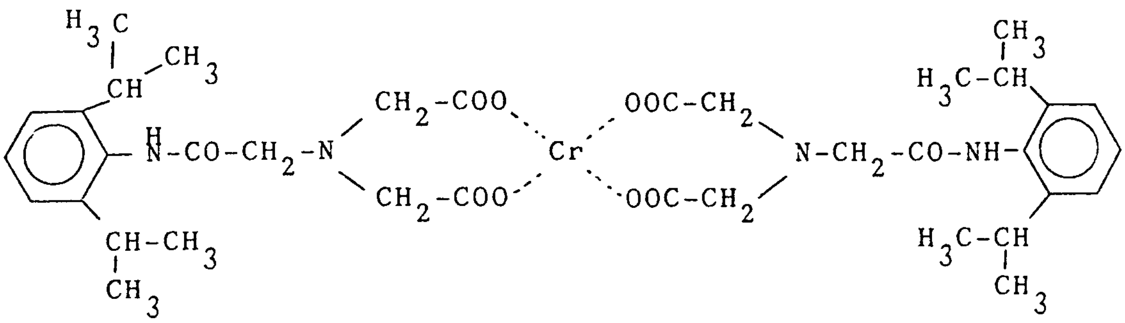

.

**12.** The compositions according to claim 7, characterized in that the target molecule and/or the connector molecule have the formula

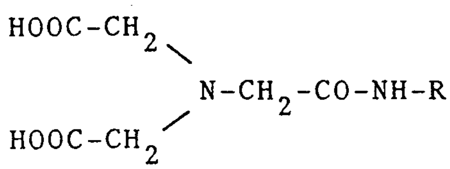 ,

wherein R is 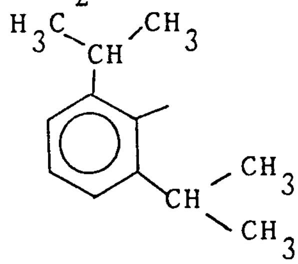 , 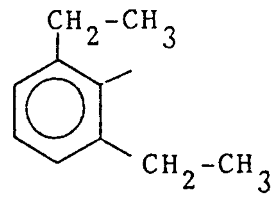 ,

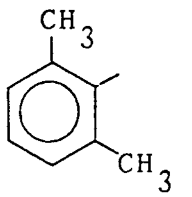 , 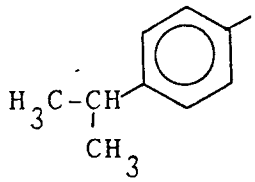 , 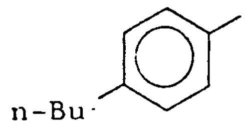 ,

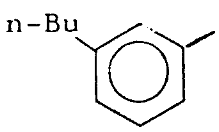 , 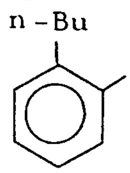 , 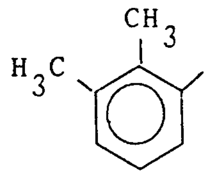 ,

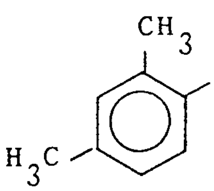 , 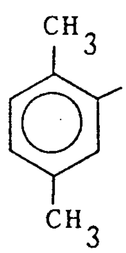 , 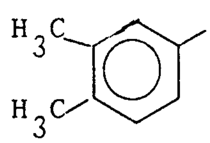 ,

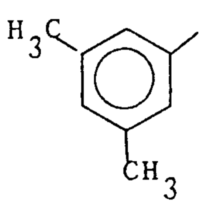 , 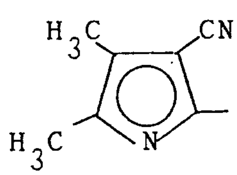 , 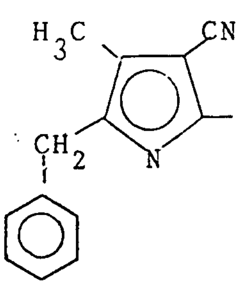 ,

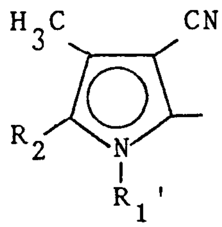 ,

| R1' | R2 |
|---|---|
| H | iso-$C_4H_9$ |
| H | $CH_2$-$CH_2$-S-$CH_3$ |
| H | $CH_2$-$C_6$-$H_4$-p-OH |
| $CH_3$ | $CH_3$ |
| $CH_3$ | iso-$C_4H_9$ |
| $CH_3$ | $CH_2$-$CH_2$-S-$CH_3$ |
| $CH_3$ | $C_6H_5$ |
| $CH_3$ | $CH_2C_6H_5$ |
| $CH_3$ | $CH_2C_6H_4$-p-O-$CH_3$ , |

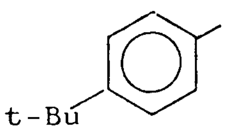 , 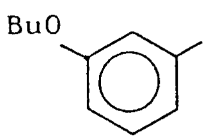 , 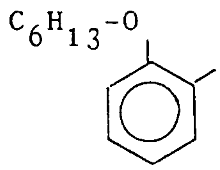 ,

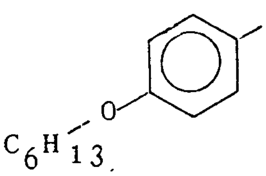 or 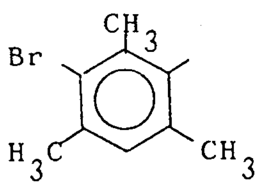 .

**13.** The compositions according to claim 7, characterized in that the target molecule and/or the connector molecule have the formula

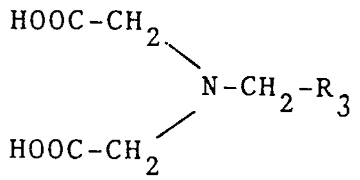

,

wherein $R_3$ is $CH_2-NH_2$,

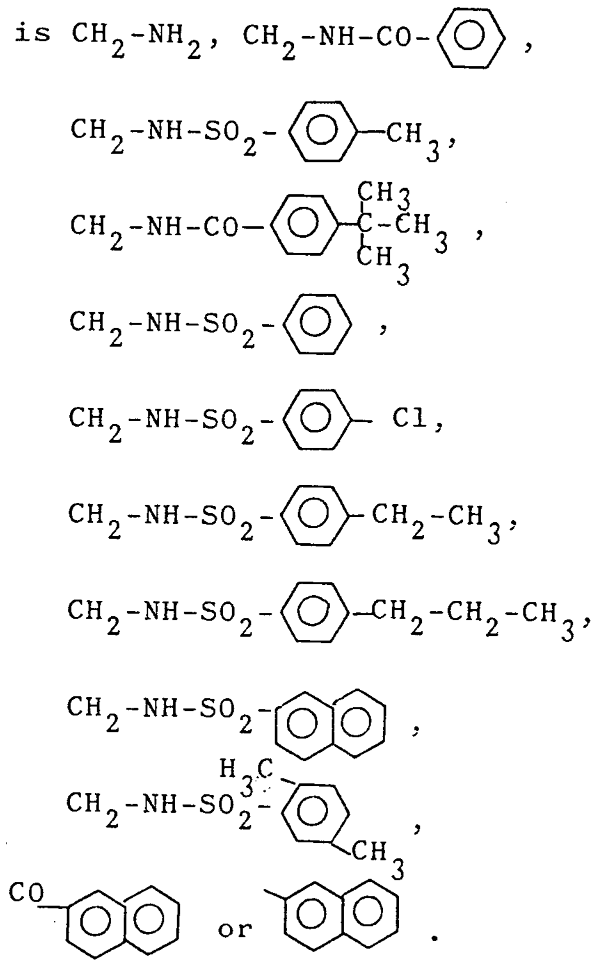

or

**14.** The compositions according to claim 7, characterized in that the target molecule and/or the connector molecule are an azo substituted iminodiacetic acid, iminodicarboxymethyl-2-naphthyl ketone, phthalein, complexone, N-(5-pregnene-3β-ol-2-oylcarbamoylmethyl)-iminodiacetic acid, 3α,7α,12α-trihydroxy-24-norcholanyl-23-iminodiacetic acid or ethylenediamine-N,N-bis(2-hydroxy-5-bromophenyl)-acetate.

**15.** The compositions according to claim 7, characterized in that the targeting molecule comprises rose bengal, congo red, bromosulphthalein, bromophenol blue, phenolphthalein, toluidine blue, indocyanine green, iodipamide, ioglycamic acid, bilirubin, cholylglycyliodohistamine, thyroxineglucuronide, penicillamine, β-mercaptoisobutyric acid, dihydrothioctic acid, 6-mercaptopurine, kethoxal-bis-(thiosemicarbazone),

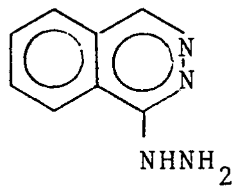

1-hydrazinophthalazine-sulfonyl urea complex, pyridoxylideneglutamate, pyridoxylidene isoleucine, pyridoxylidene phenylalanine, pyridoxylidene tryptophan, pyridoxylidene 5-methyltryptophan, 3-hydroxy-4-formyl-pyridine glutamic acid, tetracycline-7-carboxy-8-hydroxyquinoline complex, phenolphthalexon, eosin or verograffin.

**16.** The compositions according to claims 7 to 15, characterized in that the connector molecule and the target molecule are identical.

**17.** The compositions according to claims 1 to 16, characterized in that the bipolar lipid membranes comprise distearoyl lecithin, dipalmitoyl lecithin and/or other lecitins having $C_{10-20}$ chains.

**18.** The compositions according to claims 1 to 17, characterized in that the bipolar lipid membranes comprise cholesterol, dicetyl phosphate and/or albumin.

**19.** The compositions according to claims 1 to 18, characterized in that the pharmaceutical chemical is insulin.

## Revendications

**1.** Compositions consistant en ou contenant des vésicules dirigées vers les hépatocytes consistant en une membrane de lipide bipolaire renfermant un ou plusieurs produits chimiques biologiques, pharmaceutiques ou de diagnostic et comportant des molécules de ciblage fixées à la membrane de lipide et dépassant de la vésicule, caractérisées en ce que les molécules de ciblage sont douées de spécificité biologique pour les récepteurs hépatobiliaires des hépatocytes.

**2.** Les compositions selon la revendication 1, caractérisées en ce que la molécule de ciblage comprend une extrémité lipophile fixée à la structure de la membrane et une portion de ciblage douée de spécificité biologique pour les récepteurs hépatobiliaires des hépatocytes.

**3.** Les compositions selon la revendication 2, caractérisées en ce que la molécule de ciblage a une portion de ciblage de formule

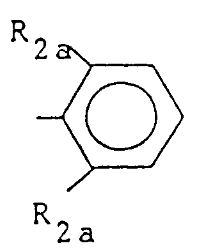

dans laquelle $R_{2a}$ est $CH_3$, $CH_3$-$CH_2$ ou $CH(CH_3)_2$.

4. Les compositions selon la revendication 2, caractérisées en ce que la molécule de ciblage a une portion de ciblage de formule

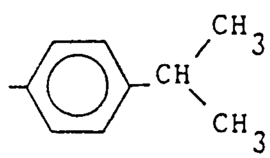

.

5. Les compositions selon les revendications 1 à 4, caractérisées en ce que la molécule de ciblage est un complexe d'iminodiacétate substitué.

6. Les compositions selon la revendication 2, caractérisées en ce que la molécule de ciblage a une portion de ciblage qui est l'acide N-(3-bromo-2,4,6-triméthylphénylcarbamoylméthyl)-iminodiacétique, l'acide N-(3-cyano-4,5-diméthyl-2-pyrrylcarbamoylméthyl)-iminodiacétique, la bilirubine ou la biliverdine.

7. Les compositions selon les revendications 2 à 6, caractérisées en ce que la molécule de ciblage est composée d'une molécule connectrice ayant une extrémité lipophile fixée à la structure de la membrane et d'une molécule cible comprenant la portion de ciblage, la molécule connectrice et la molécule cible étant liées ensemble soit directement soit au moyen d'un pont.

8. Les compositions selon la revendication 7, caractérisées en ce que la molécule connectrice et la molécule cible comprennent des extrémités terminales chargées hydrophiles reliées ensemble au moyen d'un ion de chrome, de cobalt, de fer ou de zinc.

9. Les compositions selon la revendication 7, caractérisées en ce que la molécule connectrice et la molécule cible sont reliées ensemble au moyen d'un pont de formule

$H_2N\text{-}CH_2\text{-}(CH_2)_n\text{-}NH_2$,

dans laquelle n est un nombre de 1 à 4.

10. Les compositions selon la revendication 7, caractérisées en ce que la molécule cible répond à la formule

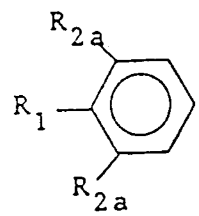

ou à la formule

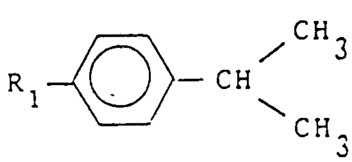

dans lesquelles $R_1$ est $[HOOC-(CH_2)_n-]_2 = N-CH_2-CO-NH-$
n étant un nombre de 1 à 3 et
$R_{2a}$ est $CH_3$, $CH_3-CH_2$ ou $CH(CH_3)_2$.

**11.** Les compositions selon les revendications 1 à 3 et 5 à 8, caractérisées en ce que la molécule de ciblage répond à la formule

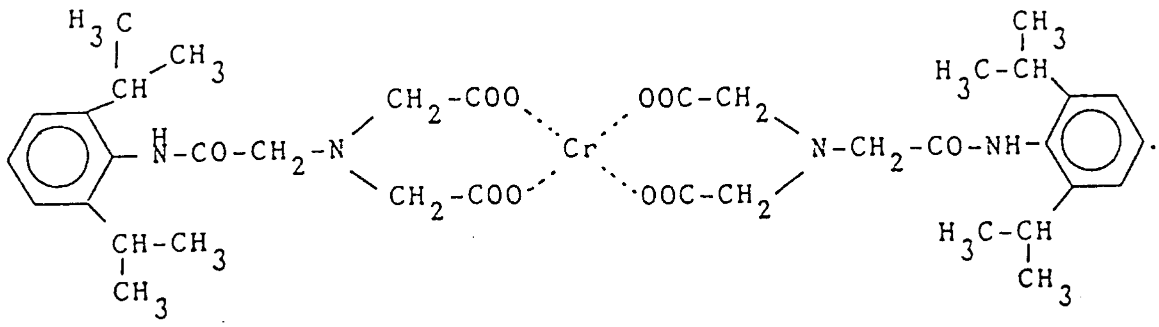

12. Les compositions selon la revendication 7, caractérisées en ce que la molécule cible et/ou la molécule connectrice répondent à la formule

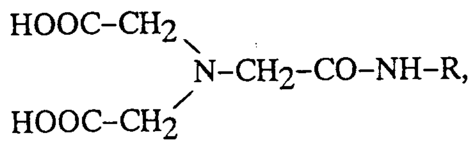

dans laquelle R est 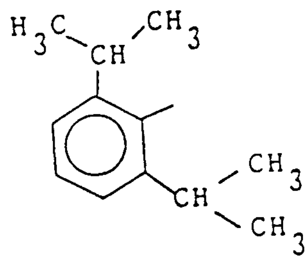 , 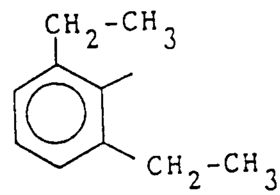 ,

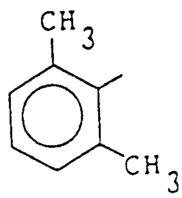 , 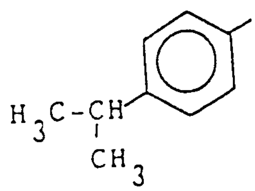 , 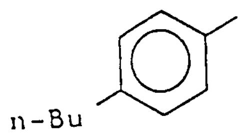 ,

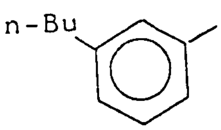 , 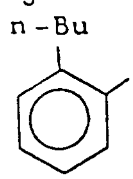 , 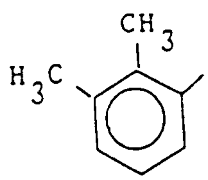 ,

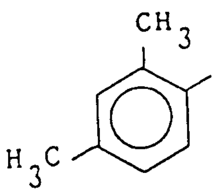 , 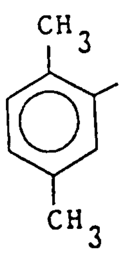 , 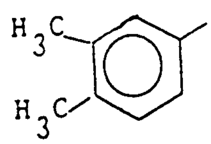 ,

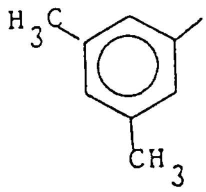 , 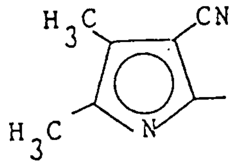 , 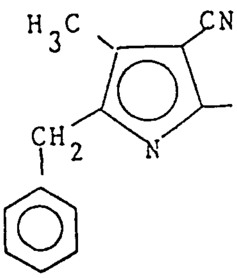 ,

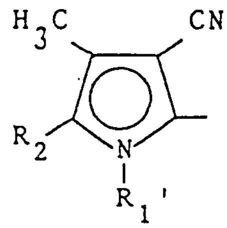 ,

| $R_1$' | $R_2$ |
|---|---|
| H | iso-$C_4H_9$ |
| H | $CH_2$-$CH_2$-S-$CH_3$ |
| H | $CH_2$-$C_6$-$H_4$-p-OH |
| $CH_3$ | $CH_3$ |
| $CH_3$ | iso-$C_4H_9$ |
| $CH_3$ | $CH_2$-$CH_2$-S-$CH_3$ |
| $CH_3$ | $C_6H_5$ |
| $CH_3$ | $CH_2C_6H_5$ |
| $CH_3$ | $CH_2C_6H_4$-p-O-$CH_3$, |

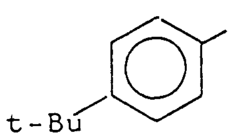 , 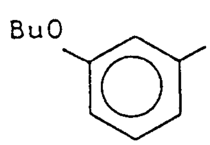 , 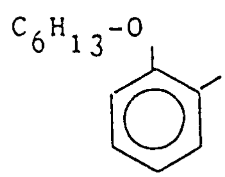 ,

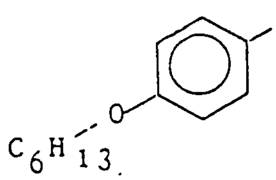 ou 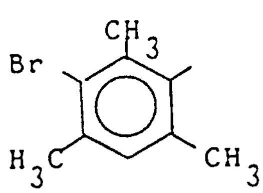 .

**13.** Les compositions selon la revendication 7, caractérisées en ce que la molécule cible et/ou la molécule connectrice répondent à la formule

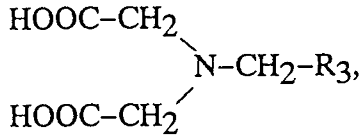

dans laquelle $R_3$ est $CH_2-NH_2$, 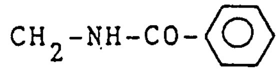 ,

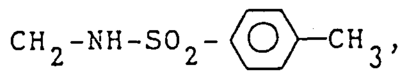

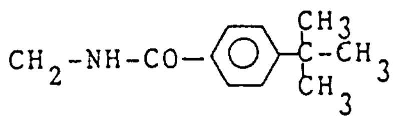

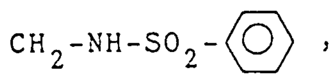

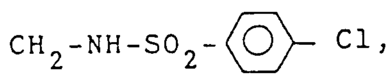

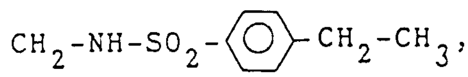

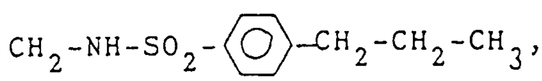

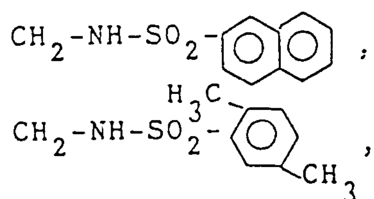

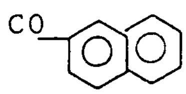 ou 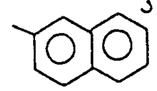 .

**14.** Les compositions selon la revendication 7, caractérisées en ce que la molécule cible et/ou la molécule connectrice sont un acide iminodiacétique azosubstitué, l'iminodicarboxyméthyl-2-naphtylcétone, la phtaléine, la complexone, l'acide N-(5-prégnène-3$\beta$-ol-2-oylcarbamoylméthyl)-iminodiacétique, l'acide 3$\alpha$,7$\alpha$,12$\alpha$-trihydroxy-24-norcholanyl-23-iminodiacétique ou un éthylène-diamine-N,N-bis(2-hydroxy-5-bromophényl)-acétate.

**15.** Les compositions selon la revendication 7, caractérisées en ce que la molécule de ciblage comprend le rose Bengale, le rouge Congo, la bromosulphtaléine, le bleu de bromophénol, la phénolphtaléine, le bleu de toluidine, le vert d'indocyanine, l'iodipamide, l'acide ioglycamique, la bilirubine, la cholylglycyliodohistamine, le thyroxineglucuronide, la pénicillamine, l'acide $\beta$-mercaptoisobutyrique, l'acide dihydrothioctique, la 6-mercaptopurine, la kéthoxal-bis(thiosemicarbazone),

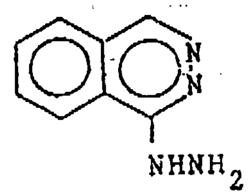

le complexe 1-hydrazinophtalazine-sulfonylurée, le pyridoxylidèneglutamate, la pyridoxylidène-isoleucine, la pyridoxylidène-phénylalanine, le pyridoxylidènetryptophane, le pyridoxylidène-5-méthyltryptophane, l'acide 3-hydroxy-4-formyl-pyridine-glutamique, le complexe tétracycline-7-carboxy-8-hydroxyquinoléine, le phénolphtalexon, l'éosine ou la verograffine.

**16.** Les compositions selon les revendications 7 à 15, caractérisées en ce que la molécule connectrice et la molécule cible sont identiques.

**17.** Les compositions selon les revendications 1 à 16, caractérisées en ce que les membranes lipidiques bipolaires comprennent de la distéaroyllécithine, de la dipalmitoyllécithine et/ou d'autres lécithines à chaînes en $C_{10}$-$C_{20}$.

**18.** Les compositions selon les revendications 1 à 17, caractérisées en ce que les membranes lipidiques bipolaires comprennent du cholestérol, du dicétyl-phosphate et/ou de l'albumine.

**19.** Les compositions selon les revendications 1 à 18, caractérisées en ce que la substance pharmaceutique est l'insuline.

**Patentansprüche**

**1.** Zusammensetzungen, die aus auf Hepatocyten gerichteten Vesikeln bestehen oder diese enthalten, wobei die Vesikel aus einer bipolaren Lipidmembran bestehen, die eine oder mehrere biologische, pharmazeutische oder diagnostische Substanzen einschließt und an die Zielerkennungsmoleküle gebunden sind, die vom Vesikel wegstehen,

**dadurch gekennzeichnet**,

daß die Zielerkennungsmoleküle biologische Spezifität für die hepatobiliären Rezeptoren von Hepatocyten besitzen.

**2.** Zusammensetzungen nach Anspruch 1, dadurch gekennzeichnet, daß das Zielerkennungsmolekül ein lipophiles Ende, das an die Membranstruktur gebunden ist, und einen Zielerkennungsteil enthält, der biologische Spezifität für die hepatobiliären Rezeptoren von Hepatocyten besitzt.

**3.** Zusammensetzungen nach Anspruch 2, dadurch gekennzeichnet, daß das Zielerkennungsmolekül einen Zielerkennungsteil der Formel

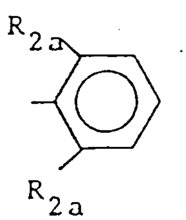

besitzt, worin $R_{2a}$ $CH_3$, $CH_3$-$CH_2$ oder $CH(CH_3)_2$ bedeutet.

4. Zusammensetzungen nach Anspruch 2, dadurch gekennzeichnet, daß das Zielerkennungsmolekül einen Zielerkennungsteil der Formel

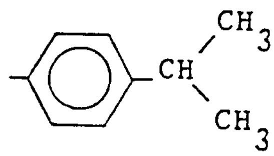

besitzt.

5. Zusammensetzungen nach den Ansprüchen 1 bis 4, dadurch gekennzeichnet, daß das Zielerkennungsmolekül ein substituierter Iminodiacetatkomplex ist.

6. Zusammensetzungen nach Anspruch 2, dadurch gekennzeichnet, daß das Zielerkennungsmolekül einen Zielerkennungsteil besitzt, der

N-(3-Brom-2,4,6-trimethylphenylcarbamoylmethyl)-iminodiessigsäure,

N-(3-Cyano-4,5-dimethyl-2-pyrrylcarbamoylmethyl)-iminodiessigsäure,

Bilirubin oder

Biliverdin

ist.

7. Zusammensetzungen nach den Ansprüchen 2 bis 6, dadurch gekennzeichnet, daß das Zielerkennungsmolekül aus einem Verknüpfungsmolekül mit einem lipophilen Ende, das an die Membranstruktur gebunden ist, und einem Zielmolekül besteht, das den Zielerkennungsteil enthält, wobei das Verbindungsmolekül und das Zielmolekül entweder direkt oder über eine Brücke miteinander verbunden sind.

8. Zusammensetzungen nach Anspruch 7, dadurch gekennzeichnet, daß das Verknüpfungsmolekül und das Zielmolekül hydrophile geladene Enden enthalten, die über ein Chromion, Cobaltion, Eisenion oder Zinkion miteinander verbunden sind.

9. Zusammensetzungen nach Anspruch 7, dadurch gekennzeichnet, daß das Verknüpfungsmolekül und das Zielmolekül über eine Brücke der Formel

$H_2N\text{-}CH_2\text{-}(CH_2)_n\text{-}NH_2$

verbunden sind, worin n 1 bis 4 bedeutet.

10. Zusammensetzungen nach Anspruch 7, dadurch gekennzeichnet, daß das Zielmolekül die Formel

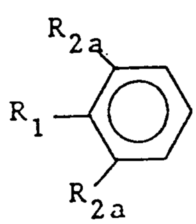

oder die Formel

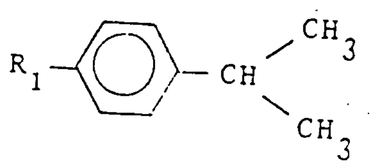

besitzt,

worin bedeuten:

$R_1$ $[HOOC-(CH_2)_n-]_2=N-CH_2-CO-NH-$ , wobei n 1 bis 3 ist,

und

$R_{2a}$ $CH_3$, $CH_3-CH_2$ oder $CH(CH_3)_2$.

**11.** Zusammensetzungen nach den Ansprüchen 1 bis 3 und 5 bis 8, dadurch gekennzeichnet, daß das Zielerkennungsmolekül die Formel

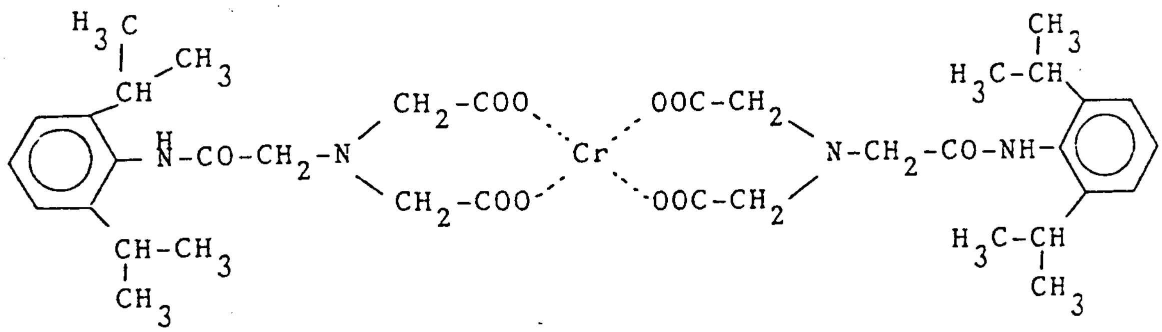

besitzt.

**12.** Zusammensetzungen nach Anspruch 7, dadurch gekennzeichnet, daß das Zielmolekül und/oder das Verknüpfungsmolekül die Formel

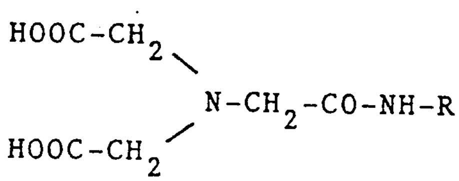

,

besitzen, worin R bedeutet:

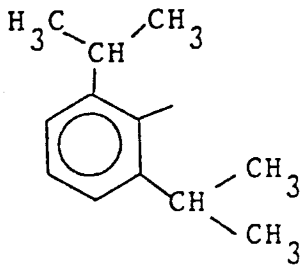 , 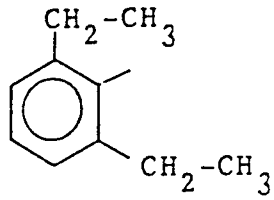 ,

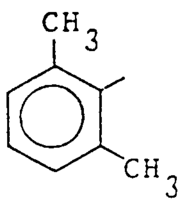 , 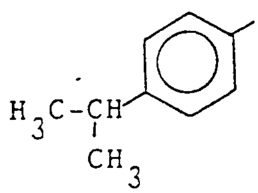 , 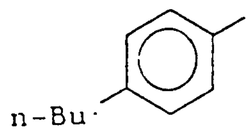 ,

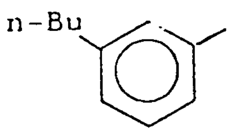 , 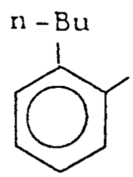 , 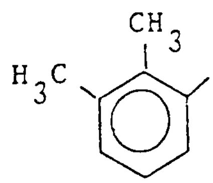 ,

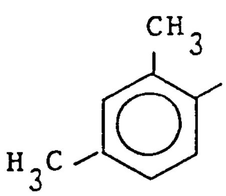 , 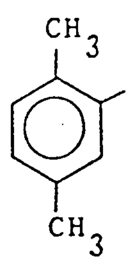 , 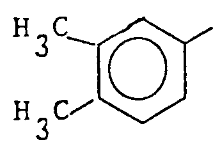 ,

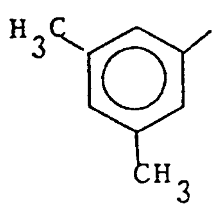 , 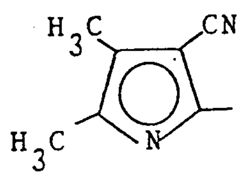 , 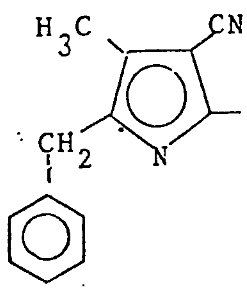 ,

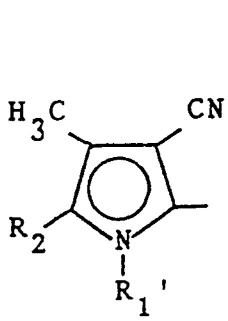 ,

| $R_1'$ | $R_2$ |
|---|---|
| H | iso-$C_4H_9$ |
| H | $CH_2$-$CH_2$-S-$CH_3$ |
| H | $CH_2$-$C_6$-$H_4$-p-OH |
| $CH_3$ | $CH_3$ |
| $CH_3$ | iso-$C_4H_9$ |
| $CH_3$ | $CH_2$-$CH_2$-S-$CH_3$ |
| $CH_3$ | $C_6H_5$ |
| $CH_3$ | $CH_2C_6H_5$ |
| $CH_3$ | $CH_2C_6H_4$-p-O-$CH_3$ , |

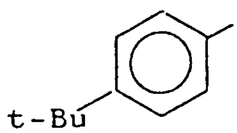
,
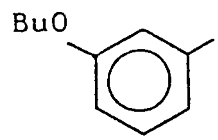
,
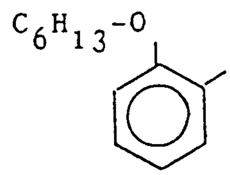
,
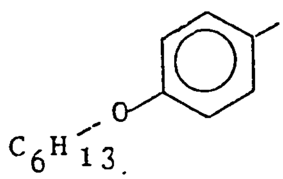
oder
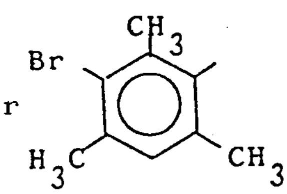
.

**13.** Zusammensetzungen nach Anspruch 7, dadurch gekennzeichnet, daß das Zielmolekül und/oder das Verknüpfungsmolekül die Formel

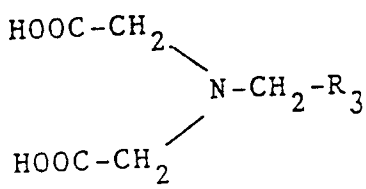

besitzen,

worin $R_3$ bedeutet:

$CH_2-NH_2$, 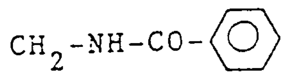 ,

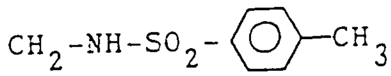 ,

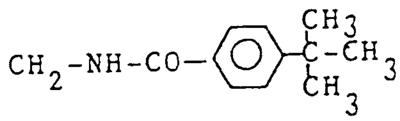 ,

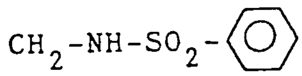 ,

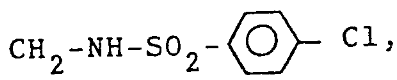 ,

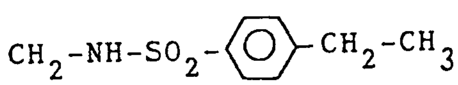 ;

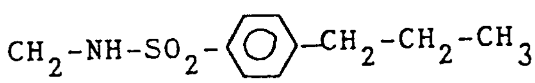 ,

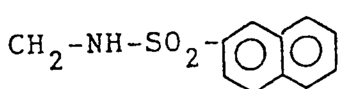 ;

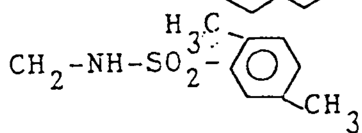 ,

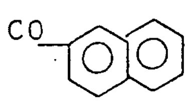 oder 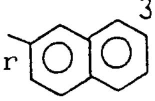 .

**14.** Zusammensetzungen nach Anspruch 7, dadurch gekennzeichnet, daß das Zielmolekül und/oder das Verknüpfungsmolekül sind: Eine azosubstituierte Iminodiessigsäure, Iminodicarboxymethyl-2-naphthylketon, Phthalein, Komplexon, N-(5-Pregnen-3$\beta$-ol-2-oylcarbamoylmethyl)-iminodiessigsäure, 3$\alpha$,7$\alpha$,12$\alpha$-Trihydroxy-24-norcholanyl-23-iminodiessigsäure oder Ethylendiamin-N,N-bis(2-hydroxy-5-bromphenyl)-acetat.

**15.** Zusammensetzungen nach Anspruch 7, dadurch gekennzeichnet, daß das Zielerkennungsmolekül eine der folgenden Verbindungen ist:
Bengalrosa, Kongorot, Bromsulphthalein, Bromphenolblau, Phenolphthalein, Toluidinblau, Indocyaningrün, Jodipamid, Joglycamsäure, Bilirubin, Cholylglycyljodhistamin, Thyroxinglucuronid, Penicillamin, $\beta$-Mercaptoisobuttersäure, Dihydrothioctansäure, 6-Mercaptopurin, Kethoxal-bis(thiosemicarbazon),

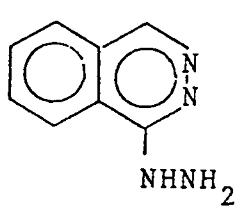 ,

1-Hydrazinphthalazin-sulfonylharnstoff-Komplex, Pyridoxylidenglutamat, Pyridoxylidenisoleucin, Pyridoxylidenphenylalanin, Pyridoxylidentryptophan, Pyridoxyliden-5-methyltryptophan, 3-Hydroxy-4-formyl-pyridinglutaminsäure, Tetracyclin-7-carboxy-8-hydroxychinolin-Komplex, Phenolphthalexon, Eosin oder Verograffin.

**16.** Zusammensetzungen nach den Ansprüchen 7 bis 15, dadurch gekennzeichnet, daß das Verknüpfungsmolekül und das Zielmolekül identisch sind.

**17.** Zusammensetzungen nach den Ansprüchen 1 bis 16, dadurch gekennzeichnet, daß die bipolaren Lipidmembranen Distearoyllecithin, Dipalmitoyllecithin und/oder andere Lecithine mit $C_{10-20}$-Ketten enthalten.

**18.** Zusammensetzungen nach den Ansprüchen 1 bis 17, dadurch gekennzeichnet, daß die bipolaren Lipidmembranen Cholesterin, Dicetylphosphat und/oder Albumin enthalten.

**19.** Zusammensetzungen nach den Ansprüchen 1 bis 18, dadurch gekennzeichnet, daß die pharmazeutische Substanz Insulin ist.

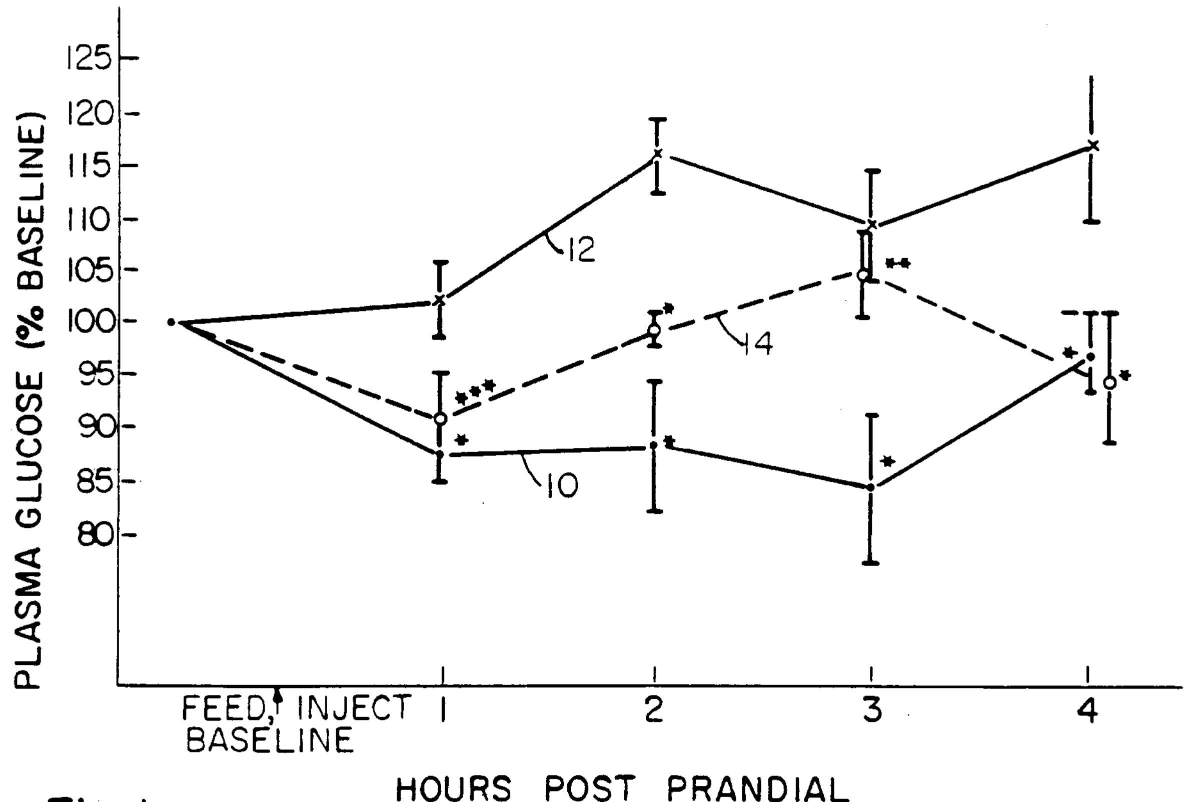
PLASMA GLUCOSE (% BASELINE)
125
120
115
110
105
100
95
90
85
80
12
14
10
FEED, INJECT
BASELINE
1
2
3
4
HOURS POST PRANDIAL

Fig. 1

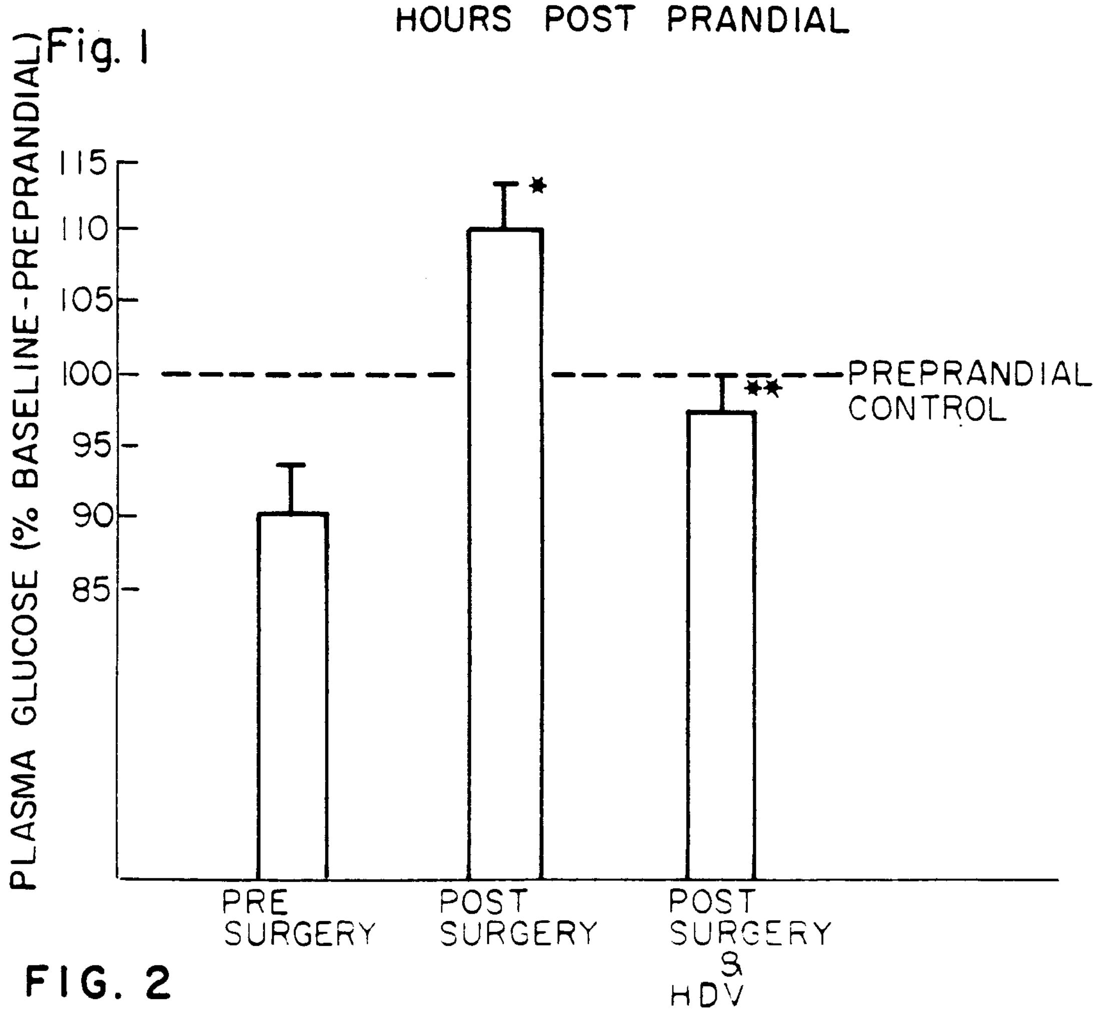
PLASMA GLUCOSE (% BASELINE-PREPRANDIAL)
115
110
105
100
95
90
85
PREPRANDIAL
CONTROL
PRE
SURGERY
POST
SURGERY
POST
SURGERY
&
HDV

FIG. 2

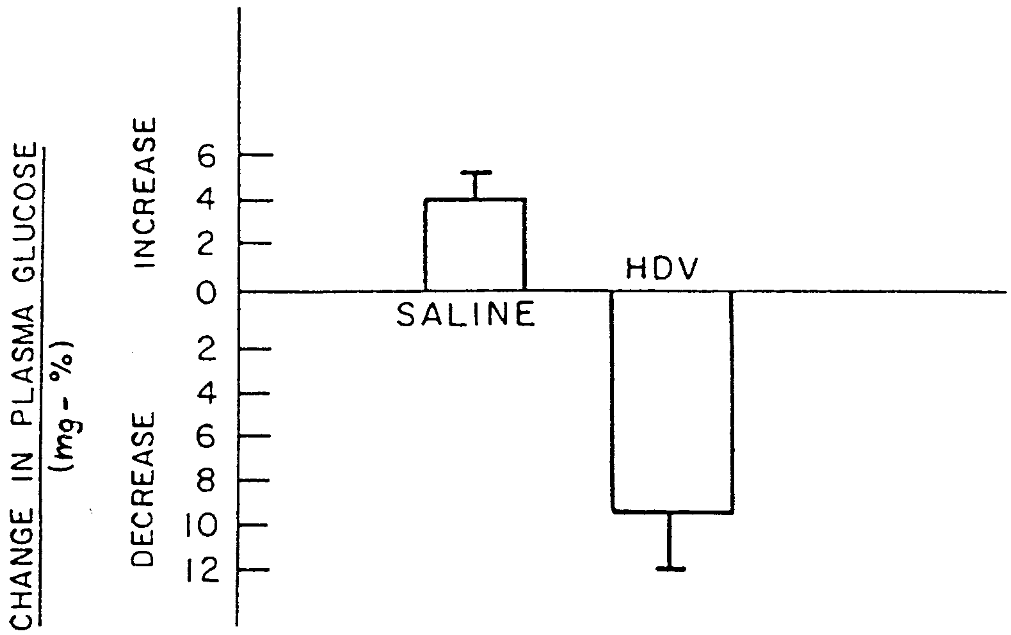
CHANGE IN PLASMA GLUCOSE
(mg - %)
INCREASE
DECREASE
6
4
2
0
2
4
6
8
10
12
SALINE
HDV

Fig. 3

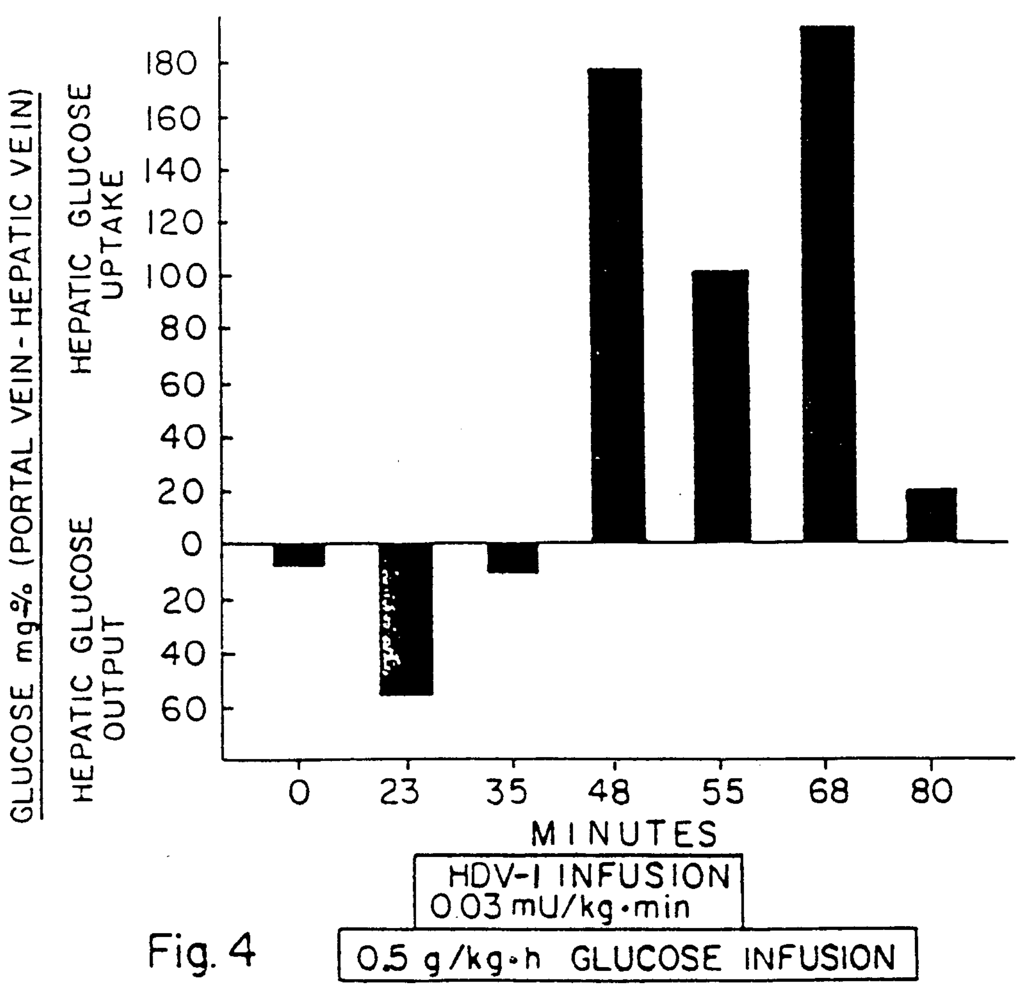
GLUCOSE mg% (PORTAL VEIN - HEPATIC VEIN)
HEPATIC GLUCOSE UPTAKE
HEPATIC GLUCOSE OUTPUT
180
160
140
120
100
80
60
40
20
0
20
40
60
0
23
35
48
55
68
80
MINUTES
HDV-I INFUSION
0.03 mU/kg·min
0.5 g/kg·h GLUCOSE INFUSION

Fig. 4

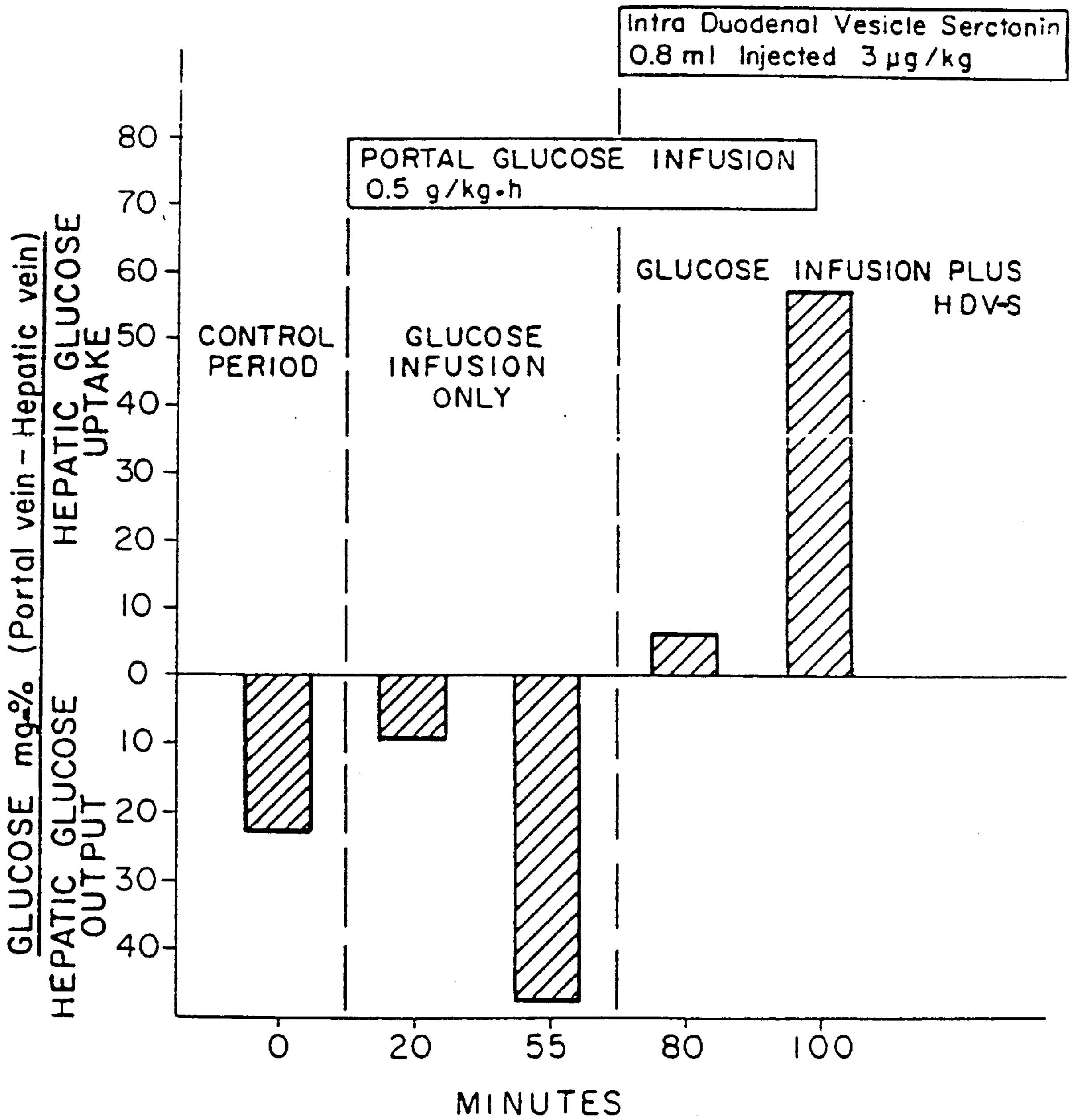

Intra Duodenal Vesicle Serctonin
0.8 ml Injected 3 μg/kg
PORTAL GLUCOSE INFUSION
0.5 g/kg·h
CONTROL PERIOD
GLUCOSE INFUSION ONLY
GLUCOSE INFUSION PLUS HDV-S
GLUCOSE mg-% (Portal vein - Hepatic vein)
HEPATIC GLUCOSE UPTAKE
HEPATIC GLUCOSE OUTPUT
80
70
60
50
40
30
20
10
0
10
20
30
40
0
20
55
80
100
MINUTES


Fig. 5

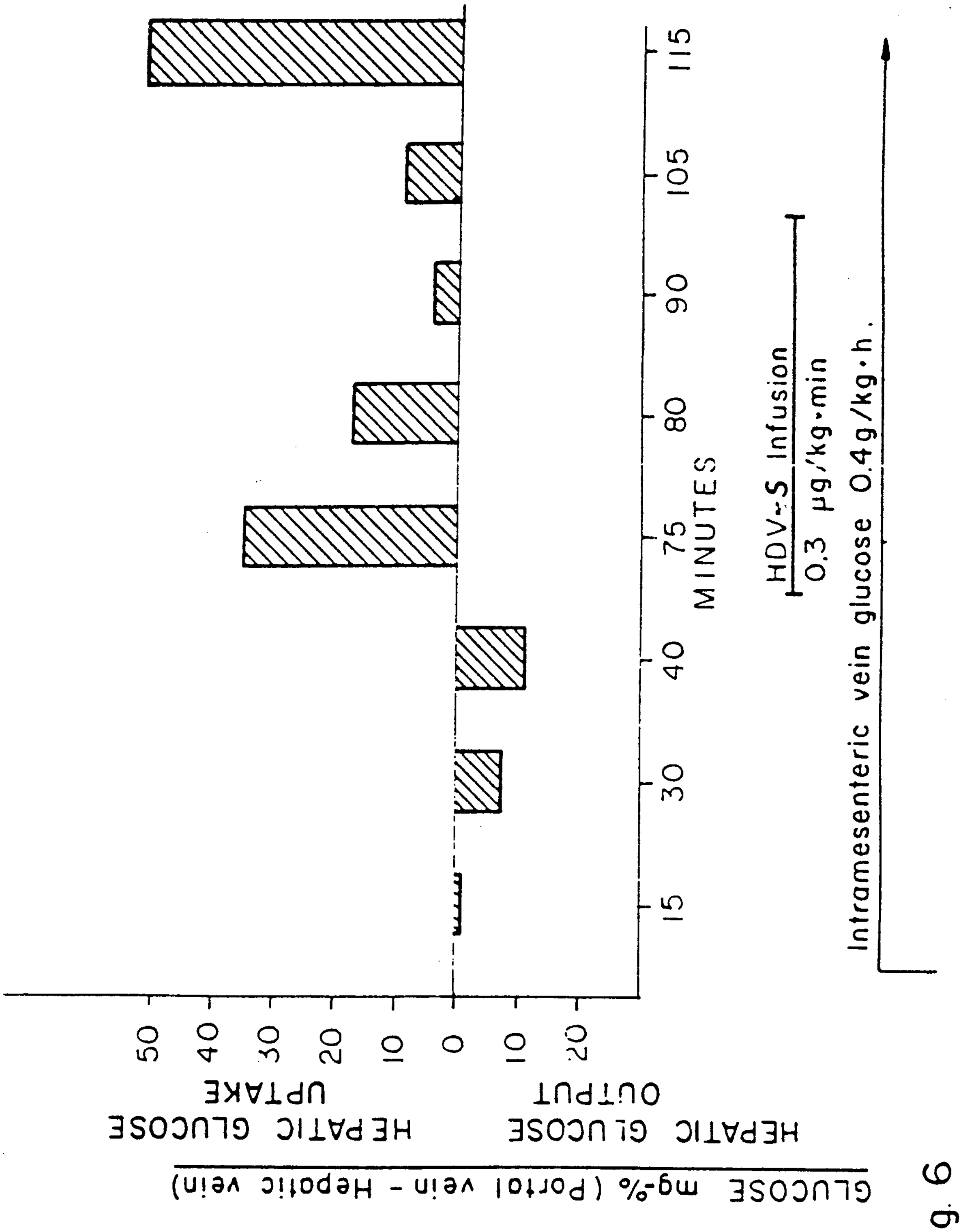
GLUCOSE mg-% (Portal vein - Hepatic vein)
HEPATIC GLUCOSE UPTAKE
HEPATIC GLUCOSE OUTPUT
50
40
30
20
10
0
10
20
15
30
40
75
80
90
105
115
MINUTES
HDV-S Infusion
0.3 µg/kg·min
Intramesenteric vein glucose 0.4g/kg·h.

Fig. 6